# EUROPEAN PATENT APPLICATION

(11) **EP 0 745 390 A2**
(43) Date of publication of application: **04.12.1996**
(21) Application number: 96108570.1
(22) Date of filing: 30.05.1996
(51) Int. Cl.: A61K 47/48

(54) **Polymeric prodrugs for beta-lactamase and uses thereof**

(30) Priority: 31.05.1995 US 19950460152
(71) Applicant: BRISTOL-MYERS SQUIBB COMPANY, Princeton, NJ 08543-4000 (US)
(72) Inventor: Senter, Peter D., Seattle, WA 98115 (US)
(74) Representative: Kinzebach, Werner, Dr., et al

(57) **Abstract**

The instant invention relates to a method for the delivery of antitumor drugs to tumor cells by the administration of a tumor-selective antibody- β-lactamase conjugate that binds to tumor cells, and the additional administration of a novel polymeric cephalosporin prodrug that is converted at the tumor site, in the presence of the antibody-β-lactamase, to an active cytotoxic drug. According to a preferred embodiment of this invention, the polymeric cephalosporin prodrug contains a polyethylene glycol or a branched polyethylene glycol moiety. The methods, antibody-enzyme conjugate, prodrugs, pharmaceutical compositions, and combinations of this invention provide for enhanced selective killing of tumor cells and are thus useful in the treatment of cancers and other tumors.

## Description

The present invention relates generally to novel prodrugs and a method for delivering these prodrugs to a tumor cell site where they are converted to active cytotoxic agents. More particularly, the invention relates to polymeric cephalosporin prodrugs, which when administered with a tumor-secific-antibody-β-lactamase conjugate, are converted at the tumor site to active cytotoxic drugs.

Targeted drug delivery systems provide a mechanism for delivering cytotoxic agents directly to cancerous cells. The selective delivery of cytotoxic agents to tumor cells is desirable because systemic administration of these agents often kills normal cells within the body as well as the tumor cells sought to be eliminated. Antitumor drug delivery systems currently in use typically utilize a cytotoxic agent conjugated to a tumor-specific antibody to form an immunoconjugate. This immunoconjugate binds to tumor cells and thereby "delivers" the cytotoxic agent to the site of the tumor. The immunoconjugates utilized in these targeting systems include antibody-drug conjugates (see, e.g., Baldwin et al., Lancet, pp. 603-605, March 15, 1986) and antibody-toxin conjugates (see, e.g., Thorpe, in Monoclonal Antibodies '84: Biological and Clinical Applications, A. Oinchera et al., eds., pp 475-506, 1985).

Both polyclonal antibodies and monoclonal antibodies have been utilized in these immunoconjugates (see, e.g., Ohkawa et al., Cancer Immunol. Immunother. 23: 81, 1986; Rowland et al., Cancer Immunol. Immunother., 21: 183, 1986). Drugs used in these immunoconjugates include daunomycin (see, e.g., Gallego et al., Int. J. Cancer, 33: 737, 1984; Arnon et al., Immunological Rev., 62: 5, 1982; mexotrexate (Endo et al., Cancer Research, 47: 1076, 1987), mitomycin C (Ohkawa et al., supra), and vindesine (Rowland et al., supra). Toxins used in the antibody-toxin conjugates include bacterial toxins such as ricin (see e.g., Moolten et al., Immunol. Rev., 62: 47, 1982).

Despite the amount of research directed towards the use of immunoconjugates for therapeutic purposes, several limitations involved in these delivery approaches have become apparent (see, e.g., Embleton, Biochem. Society Transactions, 14: 393, 615th Meeting, Belfast, 1986). For example, the large amount of drug required to be delivered to the target tumor cell to effect killing of the cell is often unattainable because of limitations imposed by the number of tumor-associated antigens on the surface of the cells and the number of drug molecules that can be attached to any given antibody molecule. This limitation has led to the use of more potent cytotoxic agents such as plant toxins in these conjugates and to the development of polymer-bound antibody-drug conjugates having very high drug multiplicity ratios (see, e.g., Thorpe, supra, pp. 475-506, and Baldwin et al., in Monoclonal Antibodies and Cancer Therapy, pp. 215-231, Alan R. Liss, Inc., 1985). However, even with the large drug loading ratios or with the use of potent toxins, many immunoconjugates still display suboptimal cytotoxic activity and are unable to effect complete killing at doses where all available antigenic sites are saturated.

It has also been recognized that the cytotoxic activity of an immunoconjugate is often dependent on its uptake, mediated by the antibody component of the conjugate into the tumor cell (see, e.g., J.M. Lambert et al., J. Biol. Chem., 260: 12035, 1985). This internalization is crucial when using an antibody-drug conjugate in which the drug has an intracellular site of action or when using antibody-toxin conjugates. However, the vast majority of tumor-associated antigens and thus the antibody-drug or antibody-toxin conjugates bound to those antigens, are not internalized. Those conjugates that are internalized are often transported to the lysosome of the cell where the drug or toxin is degraded (see Vitetta et al., Science, 238: 1098, 1987). Accordingly, although an antibody-drug or antibody toxin conjugate may have excellent tumor-binding characteristics, the conjugate may nonetheless have a limited cytotoxic utility due to an inability to reach its site of action within the cell.

In addition, it is well established that tumor cell populations are often heterogeneous with respect to antigen expression (see, e.g., Albino et al., J. Exp. Med., 154: 1764, 1981). Furthermore, it has been demonstrated that antigen-positive tumor cells may give rise to antigen-negative progeny (see, e.g., Yeh et al., J. Immunol, 126: 1312, 1981). Thus, in any population of tumor cells, there will be a certain number of cells that do not possess the antigen for which a particular immunoconjugate is specific. The immunoconjugate will therefore not be able to bind to these cells and mediate their killing.

Due to these drawbacks, the currently utilized antitumor drug or toxin delivery systems have had a limited amount of success, especially when used for in vivo treatment.

In addition to the immunoconjugates discussed above, antibody-enzyme conjugates have been studied in vitro in combination with a second untargeted enzyme for the conversion of iodide or arsphenamine to their toxic forms in order to amplify antibody-mediated cytotoxicity (see, e.g., Parker et al., Proc. Natl. Acad. Sci. USA, 72: 338, 1975; Philpott et al., Cancer Research, 34: 2159, 1974).

According to these in vitro studies, the enzyme, glucose oxidase, is attached to an antibody and used in combination with an untargeted peroxidase enzyme to convert iodide or arsphenamine to cytotoxic iodine or arsenical, respectively. This approach, therefore, requires not only the targeting of glucose oxidase to tumor cells with antibody, but also the presence at the tumor site of two other untargeted events. The likelihood that all three of these agents will be present in vivo at the tumor site at the same time is small.

Canadian Patent No. 1,216,791, discloses the conjugation to an antibody of an enzyme capable of liberating ammonium ions from substrates. The ammonium ions are then said to potentiate the cytotoxic action of certain immunotoxins targeted to the tumor site.

European Patent Application No. 84302218.7 discloses a method for treating a diseased cell population such as a tumor wherein an antibody is used to target a non-metabolizable antigen to tumor cells. The antigen accumulates within at least a percentage of the tumor cells, which are then lysed to release the antigen into a ubiquitous fibronectin capturing matrix formed at the tumor site. An iodine-containing ligand which is specific for and will bind to the antigen affixed to the matrix is administered. The cytotoxic iodine acts to kill the tumor cells at that site. Also suggested is the use of an antibody-conjugate to target enzyme to a tumor site and the addition of a non-lethal substrate which the enzyme can convert to a cytotoxic material (see European Application No. 84302218.7, pp. 34-35). However, nowhere in the application is there any disclosure of how one is perform this embodiment. Similarly, Hellstrom et al., in Controlled Drug Delivery (2d ed.), Robinson and Lee (eds.) p. 639, 1987, suggest that "drugs which would be nontoxic until activated by an agent (e.g., an enzyme) localized to a tumor may be another approach...."

U.S. Patent No. 4,975,278, hereby incorporated by reference in its entirety, provides a method for delivering cytotoxic agents to tumor cells by the combined use of antibody-enzyme conjugates and prodrugs. According to this invention, an enzyme that is capable of converting a poorly or non-cytotoxic prodrug into an active cytotoxic drug is conjugated to a tumor-specific antibody. This antibody-enzyme conjugate is administered to a tumor-bearing mammalian host and binds, due to the antibody specificity, to the surface of those tumor cells which possess the tumor antigen for which the antibody is specific. The prodrug is then administered to the host and is converted at the tumor site by the action of the antibody-bound enzyme into a more active cytotoxic drug.

Nitrogen mustards have long been recognized as cytotoxic agents (See, e.g., Stock, in Drug Design, E. J., Ariens, ed., Vol. II, pp. 532-571, Academic Press, New York, 1971.) Benn, et al., J. Chem. Soc., 2365 (1961) prepared a variety of amides, including urethanes and ureas, from N,N-di-2'-chloroethyl-para-phenylenediamine that are useful for reactions with various functional groups that are of potential value for the attachment of nitrogen mustards to a wide variety of other units. The attachment of the electron-attracting urethane group deactivates the highly toxic nitrogen mustard. Reactivation of the nitrogen mustard at the tumor site may occur if the urethane is decomposed by fission of the ester or peptide linkage.

Mobashery, et al. (J. Am. Chem. Soc., 108:1685, 1986) teaches the use of β-lactamases resident in bacteria resistant to the β-lactam antibiotics, to hydrolyze cephalosporin-toxophore derivatives to effect the release of the toxophore within the bacterium.

Mobashery et al., (J. Biol. Chem., 261: 7879, 1986) synthesized an antibacterial agent consisting of the antibiotic peptide βCl-LAla-βCl-LAla linked through a C₁₀ ester to the cephem nucleus of cephalosporin. The hydrolytic cleavage of the β-lactam ring by β-lactamase resident in the bacterium releases the heteroatom-linked C10 substituent.

A general discussion of the chemistry of the cephalosporins is provided by Abraham, Quarterly reviews - Chemical Society, 21:231, 1967, and Abraham et al., in Cephalosporins and Penicillins: Chemistry and Biology, E.H. Flynn, ed., Academic Press, N.Y, 1972, pp 1-26.

U.S. Patent No. 3,484,437 teaches derivatives of cephalosporanic acid formed by the reaction of a deacylated cephalosporin salt with isocyanates to form carbamates.

U.S. Patent No. 3,355,452 teaches the 0-desacetyl-O-carbamoyl-7-acylamino-cephalosporanic acid derivatives of 7-amino-cephalosporanic acid, where the 7-N-acyl group is a carboxylic acid radical and the CO group is bonded to a carbon atom.

There has been a great deal of investigation concerning the use of polymers as carriers of anticancer drugs (Maeda, H., et al. (1992), Bioconjugate Chem. 3, 351-362; Duncan, R. (1992), Anticancer Drugs 3, 175-210). The molecular weight carriers can lead to reductions in systemic toxicity, longer retention time in the body, alterations in biological distribution, and in improvements in therapeutic efficacy. Polyethylene glycol (PEG¹, Zalipsky, S., et al., (1983) Eur. Polym. J. 12, 1177-1183; Ouchi, T., et al., (1992), Drug Design Discovery 9, 93-105); Caliceti, P., et al., (1993), II Farmaco 48, 919-932; Panarin, E. F., et al., (1989), J. Controlled Rel. 10, 119-129), PEG copolymers (Poiani, G. J., et al., (1994), Bioconjugate Chem. 5, 621-630), dextran (Munechika, K., et al., (1994), Biol. Pharm. Bull. 17, 1193-1198), hydroxypropylmethacrylamide (Seymore, L. W., et al., (1994), Br. J. Cancer 70, 636-641), and poly(styrene-co-maleic acid) (Maeda, H, (1992), J. Controlled Rel., 19, 315-324) are but a few examples of polymers that have been used to deliver anticancer drugs and other biologically active molecules to target issues.

In most cases, release of active anticancer drugs from the polymer support is mediated by simple aqueous hydrolysis or by proteolytic or esterase enzymes (Maeda, H., et al. (1992), Bioconjugate Chem. 3, 351-362; Duncan, R. (1992), Anticancer Drugs 3, 175-210). Since the conditions for these reactions are not preferentially confined to tumor tissues, some nonspecific drug release is inevitable. Consequently, there may be advantages in developing strategies for drug release that exploit some of the physiological and biochemical differences between neoplastic and normal tissues. Such differences may either be inherent, or established by targeting enzymes to tumor cell surfaces in the form of mAb-enzyme conjugates that recognize tumor associated antigens. This targeting strategy, which has been successfully applied to the activation of a number of low molecular weight anticancer prodrugs (Bagshawe, K. D. (1994), Clin. Pharmacokinet. 27, 368-376; Deonarain, M. P., et al. (1994), Br. J. Cancer 70, 786-794; Senter, P. D., et al., (1993) Bio-conjugate Chem. 4, 3-9), should also be applicable to the release of active drugs that are covalently bound to polymer supports.

Many of the enzymes utilized for prodrug activation carry out reactions not normally occurring in mammalian systems. For example, cephalosporin containing prodrugs undergo drug elimination when hydrolyzed by β-lactams (Vrudhula, V. M., et al., (1993), Bioconjugate Chem. 4, 334-340; Meyer, D. L., et al., (1993) Cancer Res. 53, 3956-3963).

Heretofore, polymeric cephalosporin prodrugs have been unknown.

The present invention is based on the discovery of novel polymeric cephalosporin-related prodrugs, capable of conversion to antitumor agents at the tumor site using a β-lactamase-antibody conjugate. The antibody is directed against a tumor antigen present on the surface of the specific tumor type targeted.

The present invention provides polymeric cephalosporin prodrugs of the general formula (I) 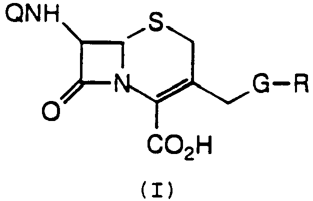 wherein Q is a pharmaceutically acceptable polymeric moiety; G is -NH- or is of the formula 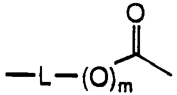 L is a direct bond or -S-(CH₂)ₙ-; R is an agent capable of exerting a cytotoxic effect on tumor cells when released from said cephalosporin-prodrug; n is 2, 3, or 4; and m is 0 or 1 with the proviso that when L is a direct bond, m is 1; or a pharmaceutically acceptable salt thereof.

It is preferred that the polymeric moiety, i.e., "Q" is the residue of a polyethylene glycol or a branched polyethylene glycol.

The cytotoxic compound is one having at least one functional group amenable to chemical modification to provide the cephalosporin prodrug. Generally, such functional groups are selected from amino, carboxyl, and hydroxyl groups such that the linkage between the cytotoxic agent and the cephalosporin component is of the carbamate, amide, ester, and carbonate types.

In one aspect, the present invention provides as one subclass of compounds of formula (I) cephalosporin prodrugs of the general formula (II) in which the cytotoxic agent is linked to the cephalosporin nucleus via carbamate or amide group 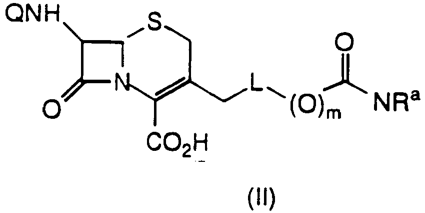 wherein Q, L, and m are as defined under formula (I); and NR^{a} is a nitrogen containing cytotoxic drug; or a pharmaceutically acceptable salt thereof.

In another aspect the present invention provides a cephalosporin-cytotoxic agent prodrug linked via an amine group such as a cephalosporin-mitomycin prodrug having the formula (IIa) 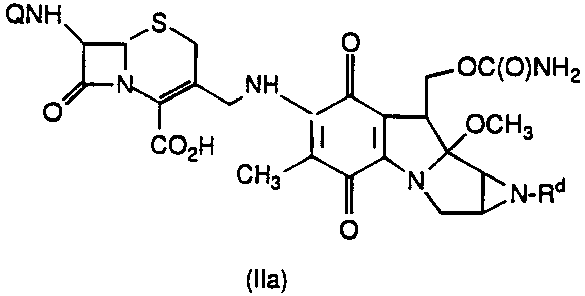 .wherein Q is as defined above under formula (I) and R^{d} is hydrogen or C₁₋₃ alkyl.

Another embodiment of the subject invention is directed to a method for delivering a cytotoxic agent to tumor cells by administering a pharmaceutically effective amount of at least one antibody-β-lactamase conjugate comprising an antibody reactive with an antigen on the surface of the tumor cells. A pharmaceutically effective amount of a polymeric cephalosporin prodrug is also administered, where the polymeric cephalosporin prodrug comprises a polymeric moiety linked in the cephalosporin which is in turn linked to the cytotoxic agent.

In an alternative embodiment, the present invention is directed to a method of delivering a cytotoxic agent to tumor cells wherein the antigen binding region of an antibody reactive with a tumor-associated antigen is linked to at least a functionally active part of β-lactamase, and is administered with a pharmaceutically effective amount of a polymeric cephalosporin prodrug.

In another embodiment, the subject invention is directed to a method of treating mammalian tumors which includes the step of administering to a mammal a pharmaceutically effective amount of at least one antibody-β-lactamase conjugate and a pharmaceutically effective amount of at least one polymeric cephalosporin prodrug.

These and other embodiments of the present invention will readily occur to those of ordinary skill in the art in view of the disclosure herein.

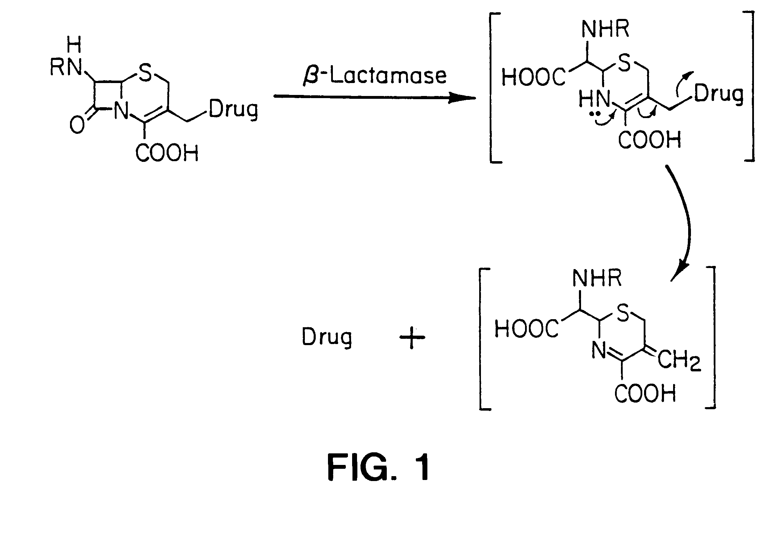

Figure 1. Mechanism of drug release by β-lactamase.

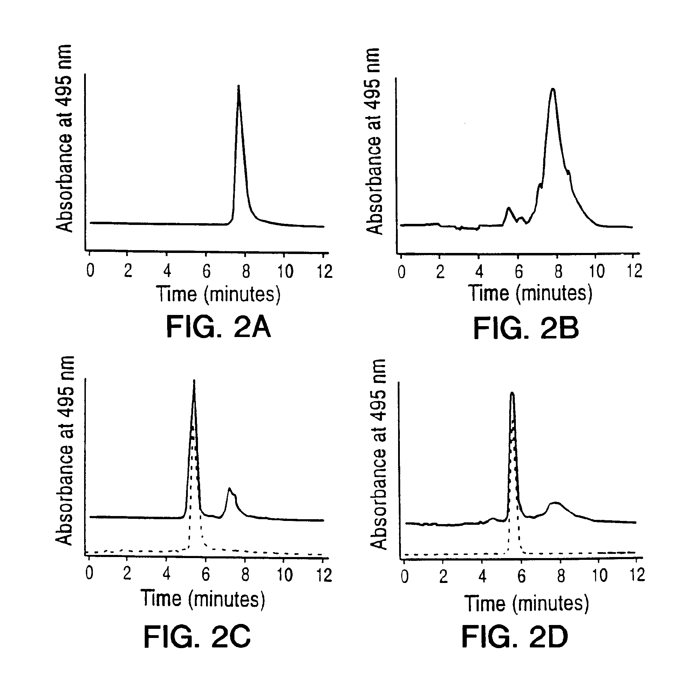

Figure 2. HPLC analyses of PEG-AC-Dox samples. A: M-PEG-AC-Dox. B: B-PEG-AC-Dox. C: M-PEG-AC-Dox + bL (solid line), Dox (dotted line). D: B-PEG-AC-Dox + bL (solid line), doxorubicin (dotted line). See the example section for the meaning of the abbreviations.

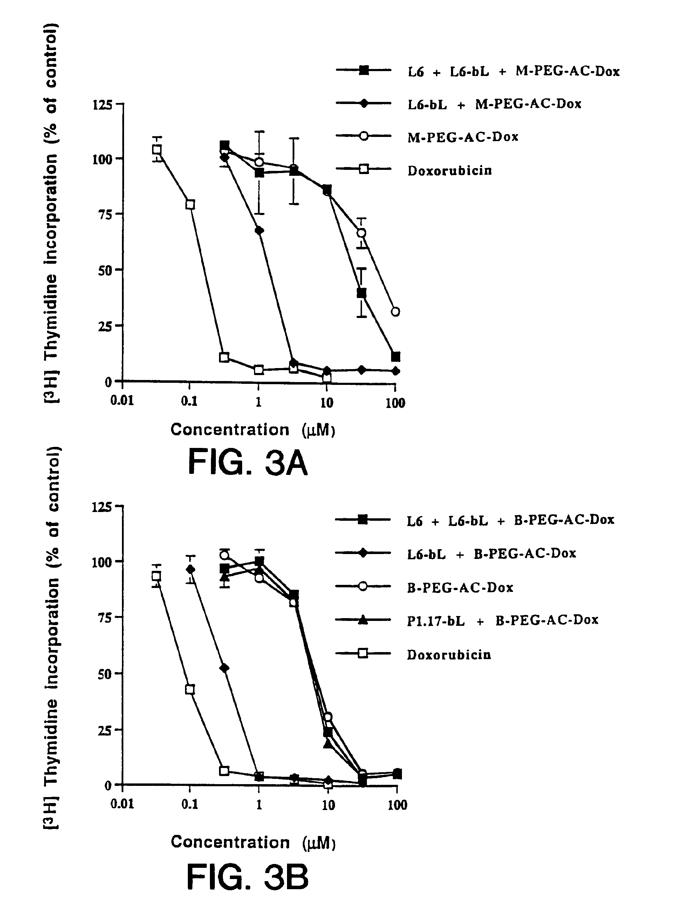

Figure 3. Cytotoxic effects of PEG-AC-Dox samples ±mAb-bL conjugates on H2981 lung adenocarcinoma cells as determined by the incorporation of [³H] thymidine into DNA compared to untreated control cells. A: Cells were exposed to L6-bL, washed and then treated with M-PEG-AC-Dox for 1 hour. The cytotoxic effects were determined approximately 24 hours later. B: Cells were exposed to conjugates, washed, and then treated with B-PEG-AC-Dox for 2 hours.

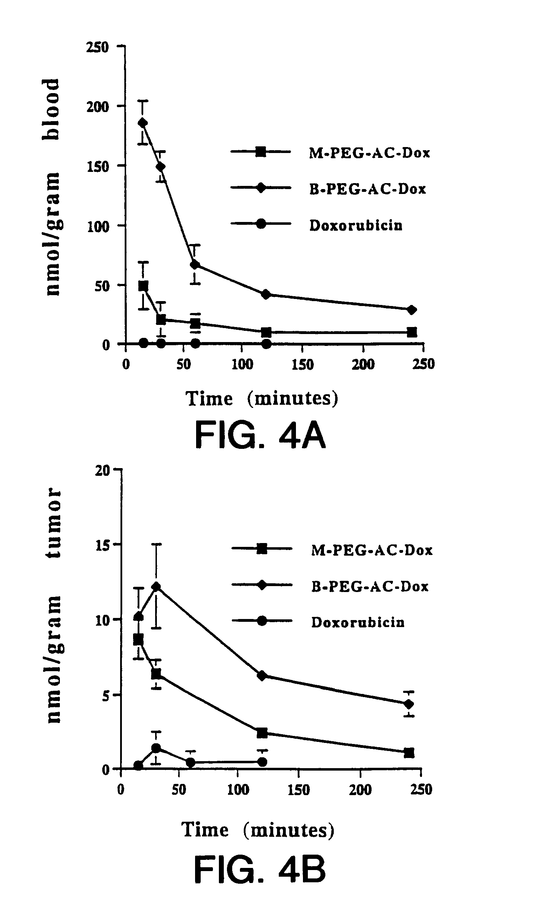

Figure 4 Pharmacokinetics of PEG-AC-Dox samples and doxorubicin in nude mice bearing subcutaneous 3677 human tumor xenografts. Tumor and blood samples were extracted, and the amount of prodrug and drug was quantified by HPLC. A: Blood levels of M-PEG-AC-Dox and B-PEG-AC-Dox after intravenous prodrug injection (52 µmol/kg). The results are compared with doxorubicin levels in animals receiving 15.5 µmol/kg doxorubicin. B: Tumor levels of M-PEG-AC-Dox, B-PEG-AC-Dox, and doxorubicin after intravenous injection (52 µmol polymer/kg, 15.5 µmol free Dox/kg).

The practice of the present invention will employ, unless otherwise indicated, conventional techniques of synthetic organic chemistry, protein chemistry, molecular biology, microbiology, and recombinant DNA technology, which are within the skill of the art. Such techniques are explained fully in the literature. See, e.g., Scopes, R.K., Protein Purification Principles and Practices, 2d ed. (Springer-Verlag, 1987), Methods in Enzymology (S. Colowick and N. Kaplan, eds., Academic Press, Inc.), Sambrook et al., Molecular Cloning: A Laboratory Manual, 2d ed., Cold Spring Harbor Press, Cold Spring Harbor, NY, 1989, Handbook of Experimental Immunology, Vols. I-IV (D.M. Weir and C.C. Blackwell, eds, 1986, Blackwell Scientific Publications); House, Modern Synthetic Reactions, 2nd ed., Benjamin /Cummings, Menlo Park, Cal., 1972.

All patents, patent applications, and publications mentioned herein, whether supra or infra, are hereby incorporated by reference in their entirety.

### A. Definitions

In defining the present invention, the following terms will be employed, and are intended to be defined as indicated below.

The term "prodrug" as used in this application refers to a precursor or derivative form of a pharmaceutically active substance that is less cytotoxic to cells compared to the parent drug and is capable of being enzymatically activated or converted into the more active parent form. See, e.g, Wilman, Biochem. Society Transactions, 14:375 (615th Meeting, Belfast, 1986); Stella et al., Directed Drug Delivery, R. Borchardt et al., ed., 247-267 (Humana Press, 1985). The terms "parent drug", "drug" and "cytotoxic agent" are used interchangeably herein.

The term "cephalosporin prodrug" as used herein refers to a prodrug generated by the linkage of a parent compound as described above to a cephalosporin as defined below.

The term "β-lactamase" as used herein refers to any enzyme capable of hydrolyzing the CO - N bond of a β-lactam ring. The β-lactamases are reviewed in Bush, Antimicrobial. Agents Chemother., 33:259, 1989.

The term "nitrogen mustard" as used herein refers to a compound of the general structure RN(CH₂CH₂Cl)₂, where R may be an alkyl, aryl, or aralkyl group substituted with a functional group amenable to further chemical modification, for example, an amino or a carboxyl group. Nitrogen mustards having more than one nitrogen atom are also included, such that both chloroethyl groups need not be attached to the same nitrogen atom. In some nitrogen mustards, the chlorine atoms may be replaced with other halogen atoms, especially bromine, or mesylates. See, e.g., Stock, in Drug Design, E. J., Ariens, ed., Vol. II, pp. 532-571, Academic Press, New York, 1971.

The term "cephalosporin" as used herein refers to derivatives of 7-aminocephalosporanic acid having the characteristic β-lactam dihydrothiazine ring of cephalosporin C, occurring either naturally or synthetically. Examples of these derivatives and a review of the chemistry of the cephalosporins is given in Abraham, Quarterly reviews - Chemical Society, 21: 231, 1967. The term "cephem" is sometimes used herein to refer to a cephalosporin. The structure of cepha-losporin C is shown below:

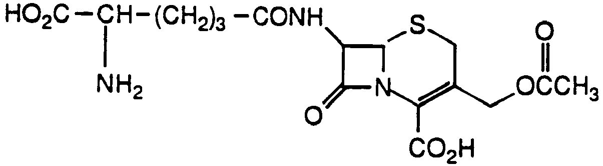

The term "cephalosporin mustard" as used herein refers to a cephalosporin as described above, wherein the cephalosporin has been derivatized with a nitrogen mustard as described above.

The term "cytotoxic" as used herein refers to the property of causing cell growth retardation or cell death, particularly as measured by a colony inhibition assay or ³H-thymidine uptake assay (see, eg., Hellstrom et al., in In Vitro Meth-ods in Cell-Mediated Immunity, Bloom and Glade, eds., 1971, and the examples herein).

The term "pharmaceutically acceptable polymeric moiety" refers to the polymeric residues of a polymer that is found upon reaction of a polymer with a cephalosporin or cephalosporin prodrug, wherein the polymeric moiety does not result in any substantial side effects and does not substantially interfere with the efficacy of the cytotoxic agents.

The term "polymeric residue" refers to the portion of the polymer that becomes covalently bonded to the 7-nitrogen of the cephalosporin ring.

### B. General Methods

The present invention relates to a novel method for the delivery of cytotoxic agents to tumor cells and provides for enhanced selective killing of tumor cells in the treatment of cancers, such as carcinomas and melanomas, as well as other tumors.

According to the method of the invention, an antibody-enzyme conjugate is administered to a tumor-bearing mammalian host. This antibody-enzyme conjugate consists of a tumor-selective antibody linked to a β-lactamase that is capable of converting a prodrug that is less cytotoxic to cells than the parent drug into the more active parent drug. When introduced into the host, the antibody component of the conjugate, which is reactive with an antigen found on the tumor cells, directs the conjugate to the site of the tumor and binds to the tumor cells. The antibody thus delivers the enzyme to the site of the tumor. A prodrug that is a substrate for the β-lactamase is also introduced into the host and is converted, at the tumor site, by the enzyme into an active cytotoxic drug. The drug is thus activated extracellularly and can diffuse into all of the tumor cells at that site, i.e., those cells bearing the particular tumor antigen to which the antibody of the conjugate is specific and to which the antibody has bound as well as those cells that are negative for that antigen but are nonetheless present at the site of the tumor. The method of this invention therefore overcomes the current problems of tumor antigen heterogeneity and the requirement of antigen/conjugate internalization associated with conventional immunoconjugate drug delivery techniques.

Furthermore, because the present method does not require the drug to be bound directly to the antibody and thereby limit the amount of drug that can be delivered, the common-place problem of drug potency at the tumor site does not arise. In fact, the present method amplifies the number of active drug molecules present at the tumor site because the antibody-bound enzyme of the conjugate can undergo numerous substrate turnovers, repeatedly converting prodrug into active drug. Moreover, the present method is capable of releasing the active drug specifically at the tumor site as opposed to release to other tissues. This is so because the concentration of the enzyme at the tumor site is higher than its concentration at other tissues due to the coating of the tumor cells with the antibody-enzyme conjugate.

The antibody of the immunoconjugate of the invention includes any antibody which binds specifically to a tumor-associated antigen. Examples of such antibodies include, but are not limited to, those which bind specifically to antigens found on carcinomas, melanomas, lymphomas, and bone and soft tissue sarcomas as well as other tumors. Antibodies that remain bound to the cell surface for extended periods or that are internalized very slowly are preferred. These antibodies may be polyclonal or preferably, monoclonal, may be intact antibody molecules or fragments containing the active binding region of the antibody, e.g., Fab or F(ab')₂, and can be produced using techniques well established in the art. See, e.g., R.A. DeWeger et al., Immunological Rev., 62:29-45, 1982 (tumor-specific polyclonal antibodies produced and used in conjugates): Yeh et al., Proc. Natl. Acad. Sci. USA, 76:2927, 1979; Brown et al., J. Immun., 127:539, 1981 (tumor-specific monoclonal antibodies produced); and Mach et al., in Monoclonal Antibodies for Cancer Detection and Therapy, R.W. Baldwin et al., eds., pp 53-64, Academic Press, 1985 (antibody fragments produced and used to localize tumor cells). In addition, if monoclonal antibodies are used, the antibodies may be of mouse or human origin or chimeric antibodies (see, e.g., Oi, Biotechniques, 4:214, 1986).

Examples of antibodies which may be used to deliver the β-lactamase to the tumor site include, but are not limited to, L6, an IgG2a monoclonal antibody (hybridoma deposit no. ATCC HB8677) that binds to a glycoprotein antigen on human lung carcinoma cells (Hellstrom, et al., Proc. Natl. Acad. Sci. USA, 83:7059, 1986); 96.5, an IgG2a monoclonal antibody that is specific for p97, a melanoma-associated antigen (Brown, et al., J. Immunol. 127:539, 1981); 1 F5, an IgG2a monoclonal antibody (hybridoma deposit no. ATCC HB9645) that is specific for the CD-20 antigen on normal and neoplastic B cells (Clark et al., Proc. Natl. Acad. Sci. USA, 82:1766, 1985).

An alternative strategy is to use antibodies that internalize, providing that the prodrug can also internalize, or that a sufficient amount of antibody also remains on the surface of the cell. An example of such antibodies may be found in Cancer Research 56:2183 (1990).

The enzyme component of the immunoconjugate of the invention includes any enzyme capable of hydrolyzing the CO - N bond of a β-lactam. Some of these enzymes are available commercially, such as E. coli or B. cereus β-lactamases. These and other β-lactamases may be cloned and expressed using recombinant DNA techniques well known in the art.

The β-lactamases of this invention can be covalently bound to antibodies by techniques well known in the art such as the use of the heterobifunctional crosslinking reagents SPDP (N-succinimidyl-3-(2-pyridyldithio)propionate) or SMCC (succinimidyl 4-(N-maleimidomethyl) cyclohexane-1-carboxylate (see, e.g., Thorpe et al., Immunol. Rev., 62: 119, 1982; Lambert et al., supra, at p. 12038; Rowland et al., supra, at pp 183-184; Gallego et al., supra, at pp. 737-7138). Alternatively, fusion proteins comprising at least the antigen binding region of an antibody linked to at least a functionally active portion of a β-lactamase can be constructed using recombinant DNA techniques well known in the art (see, e.g., Neuberger et al., Nature, 312:604, 1984). These fusion proteins act in essentially the same manner as the antibody-enzyme conjugates described herein.

The prodrugs of the invention contain an antitumor agent linked to a polymeric cephalosporin or polymeric cephalosporin derivative. The antitumor agent is activated or otherwise converted into a more active form upon cleavage of the prodrug with β-lactamase. In the preferred embodiment, the antitumor agent is a nitrogen mustard, as defined above. A representative nitrogen mustard is shown below:

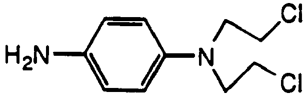

Other preferred antitumor agents include doxorubicin, which has the general formula: 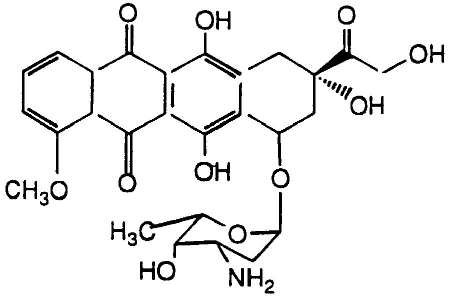 and mitomycin C, which has the general formula:

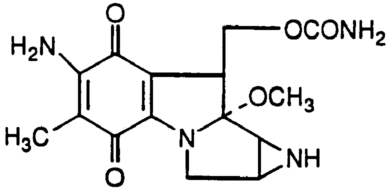

The prodrugs of this invention are not limited to these compounds, and may include other antitumor agents that can be derivatized into a prodrug form for use in a cephalosporin conjugate. Such antitumor agents include etoposide, teniposide, daunomycin, carminomycin, aminopterin, dactinomycin, cis-platinum and cis-platinum analogues, bleomycins, esperamicins (see U.S. Patent 4,675,187), and 5-fluorouracil.

In one preferred embodiment of this invention, an anthracycline-cephalosporin prodrug is synthesized by reaction of an anthracycline with a carboxyl protected 3-[(carbonyloxy)methyl]cephem such as the diphenylmethyl esters of 3-[[(p-nitrophenoxy) carbonyloxy]methyl]cephem and 3-(1,2,2,2-tetrachloroethoxy)carbonyloxy]methyl]cephem. The resulting prodrug contains an anthracycline linked to the cephalosporin by the amino group of the former through a carbamate bond.

In another preferred embodiment of this invention, a cephalosporin mustard is synthesized by reaction of a 3-hydroxymethyl cephalosporin salt with an isocyanate, as described in U.S. Patent Nos. 3,355,452, and 3,484,437, and Belgian Patent No. 741,381, herein incorporated by reference in their entirety. Such a reaction is also described in detail in the examples.

More generally, the present invention provides cephalosporin prodrugs of the general formula (I) 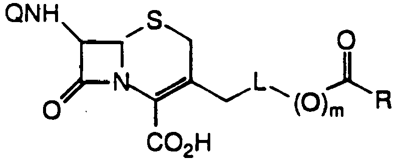 wherein Q is a pharmaceutically acceptable polymeric moiety; L is a direct bond or -S-(CH₂)ₙ-; R is an agent capable of exerting a cytotoxic effect on tumor cells when released from said cephalosporin-prodrug; n is 2, 3, or 4; and m is 0 or 1 with the proviso that when L is a direct bond, m is 1; or a pharmaceutically acceptable salt thereof.

The pharmaceutically acceptable polymeric moiety can be any of the polymeric moieties known in the art to be capable of being linked to the cephalosporin. Examples include polyethylene glycol, a branched polyethylene glycol, a polyethylene glycol copolymer, dextran, hydroxypropylmethacrylamide, poly(styrene-co-maleic acid), polygalacturonic acid, carboxymethylated β-D-glucan, N-(2-hydroxypropyl)methacrylamide copolymer, N-(2-hydroxyethyl)methacrylate-vinyl-pyrrolidone copolymer, poly(hydroxyethyl-L-glutamine), poly(α-maleic acid), a polyamino acid, and a block copolymer of poly(ethyleneimine) and palmitic acid. Such moieties are disclosed in, for example, S. Zalipsky, et al., Eur. Polym. J., 19 (12): 1177-1183, 1983; D. Caliceti, et al., II Farmaco, 48(7): 919-932, 1993; R. Duncan, Anti-Cancer Drugs, 3: 175-210, 1992; and E.F. Panarin et al., J. of Controlled Release, 10: 119-129, 1989.

The Q substituent is preferably the residue of a polyethylene glycol or a branched polyethylene glycol.

Examples of polyethylene glycols have the structure

R'-(OCH₂-CH₂)ₙ-OH

wherein R' is H or a C₁-C₄ alkyl and n is an integer of 4 to 1000. It is preferred that R¹ is H or methyl and it is preferred that n is 10 to 400, most preferred is 50 to 200. Such polyethylene glycols are commercially available and can be purchased from Shearwater Polymers, Inc., Huntsville, AL.

Examples of branched polyethylene glycols have the structure

R"-[(OCH₂-CH₂)ₙ-OH]ₓ

wherein R" is a hydrocarbon backbone containing 2 to 12 carbon atoms that is the residue of a polyalcohol,
n is an integer of 4 to 500, and
x is an integer of 2 to 12,

It is preferred that n is an integer 10 to 400, more preferred is 50 to 200. It is preferred that x is an integer of 4 to 10. The branched polyethylene glycols used to prepare the polymeric cephalosporin prodrug of the invention can be described as carbon-containing backbones or cores, e.g.divinyl benzene, having attached thereto multi-polyethylene glycol moieties.

Such branched polyethylene glycols are commercially available and can be purchased from Shearwater Polymers, Inc., Huntsville, AL.

The above described polyethylene glycol or branched polyethylene glycol is optionally preferably modified in a manner to allow facile reaction with an amine (i.e., 7-nitrogen of the β-lactam). The polyethylene glycol or branched polyethylene glycol is linked to the cephalosporin moiety via a hydroxyl group or a hydroxyl group as so modified. Modifications of polyethylene glycols or branched polyethylene glycols to allow ease of reaction with amines is well known in the art. For example, the polyethylene glycol or branched polyethylene glycol can be modified by preparing an appropriate acid chloride, mixed aldehyde or active ester (e.g., N-hydroxy succinamide ester) of the polyethylene glycol or branched polyethylene glycol (see, for example, Zalipsky S. et al., (1983) Eur. Polym. J. 12, 1177-1183). Such modifications can sometimes be termed "activations". For example, a proprionic ester can be prepared as described in the Example section. First, a propionic derivative of PEG is prepared having the formula

PEG-O-CH₂-CH₂-COOH

wherein PEG is the remaining polyethylene glycol or branched polyethylene glycol without the reacted hydroxyl moiety.

The above modified PEG polymer can then be further modified by reacting with, for example, dicyclohexylcarbodiimide and N-hydroxysuccinimide to form a PEG succinimidyl propionate compound which can then be linked with an ester linkage to the amine (7-nitrogen) of the cephalosporin moiety. Examples of PEG succinimidyl propionate compounds useful herein include the following structures:

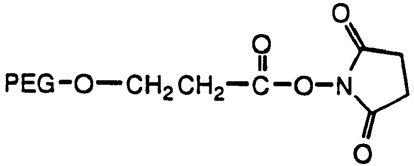

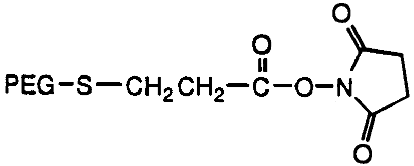

Another example of modified or activated PEG is carbonyldimidazole-activated PEG (CDI-PEG). An example of such an CDI-PEG has the formula 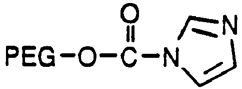 CDI-PEGs are linked to the amino group (7-nitrogen) of cephalosporins via a urethane linkage as depicted below:

CDI-PEG + NH₂-cephalosporin/drug → PEG-O₂-C-NH-cephalosporin/drug

(SEE Beauchamp, C.O. et al. (1983) Anal. Biochem. 131:25; Allen, T.M. et al. (1991) Biochem. Biophys. Acta. 1066: 29). Another example of modified PEG is PEG tresylate such of the formula:

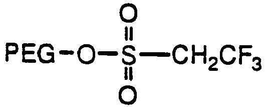

Such an electrophilically activated PEG can be linked to the cephalosporin/drug conjugate as illustrated below: 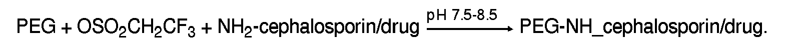 (See Nilsson, K. et al. (1984) Methods in Enzymology, 104:5-6; Klibanov, A.L., et al. (1991) Biochem. Biophys. Acta, 1062: 147)

Another electrophically activated PEG is an epoxide, an example of which has the structure:

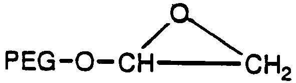

Such epoxides can be linked to the cephalosporin/drug conjugate as illustrated below: 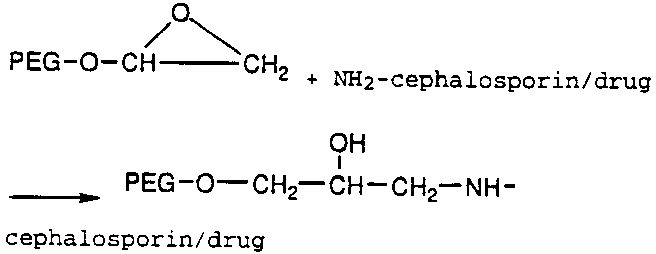 (See Pitha, J., et al. (1979) Eur. J. Biochem. 94:11; Bergström, K. et al. (1992), J. Biomed Mat. Res. 26: 779)

Other examples of modified PEG useful herein have the following structures: 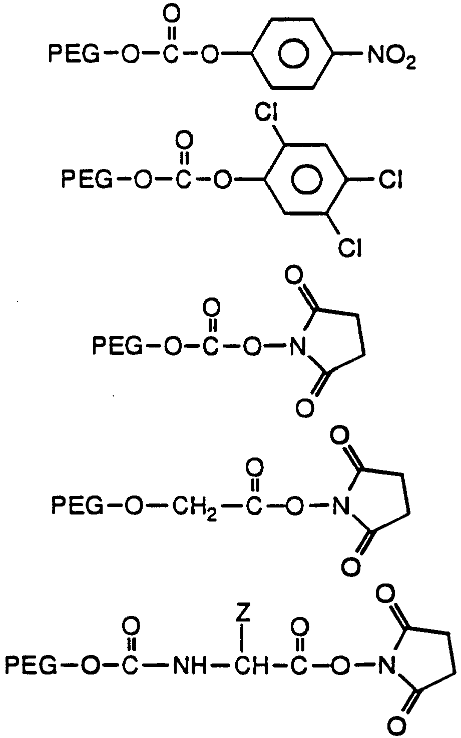 wherein Z is the side chain of an amino acid

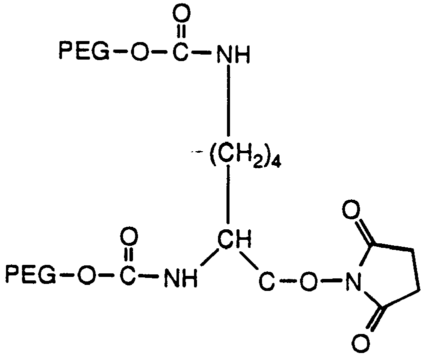

The cytotoxic compound is one having at least one functional group amenable to chemical modification to provide the cephalosporin prodrug. Generally, such functional groups are selected from amino, carboxyl, and hydroxyl groups such that the linkage between the cytotoxic agent and the cephalosporin component is of the carbamate, amide, ester, and carbonate types.

In one aspect, the present invention provides as one subclass of compounds of formula (I) polymer cephalosporin prodrugs of the general formula (II) in which the cytotoxic agent is linked to the cephalosporin nucleus via carbamate or amide group 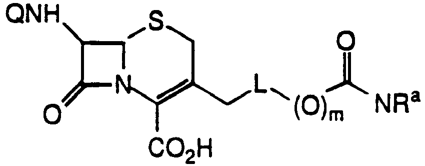 wherein Q, L, and m are as defined under formula (I); and NR^{a} is a nitrogen containing cytotoxic drug; or a pharmaceutically acceptable salt thereof.

Compounds of formula (II) before linkage of the polymeric moiety wherein L is a direct bond may be prepared by reaction sequences illustrated in Scheme I. Thus, a 3-hydroxymethyl cephalosporin (III), preferably in an alkali metal salt form such as the sodium or potassium salt, is reacted with an isocyanato derivative of a cytotoxic agent in the presence of a tertiary amine base in an aprotic solvent to afford compounds of formula (V). Alternatively, a carboxyl-protected cephalosporin carbonate of formula (IV) is treated with a nitrogen containing cytotoxic agent followed by deprotection of the carboxyl group to provide the desired cephalosporin prodrug (V). The cephalosporin carbonate (IV) may, in turn, be prepared from a carboxyl-protected 3-hydroxymethyl cephalosporin upon reaction with a chloroformate, e.g., 4-nitrophenyl chloroformate and 1,2,2,2-tetrachloroethyl chloroformate. In Scheme I, and NR^{a} have the same meaning as defined under formulas (I) and (II), R¹ is an ester activating group, preferably 4-nitrophenyl, or 1,2,2,2-tetrachloroethyl; and R² is a carboxyl protecting group, for example, benzyl, t-butyl, diphenylmethyl, allyl and the like. The carboxyl protecting group may be removed using conventional techniques such as acid catalyzed hydrolysis and reductive palladium catalysis. 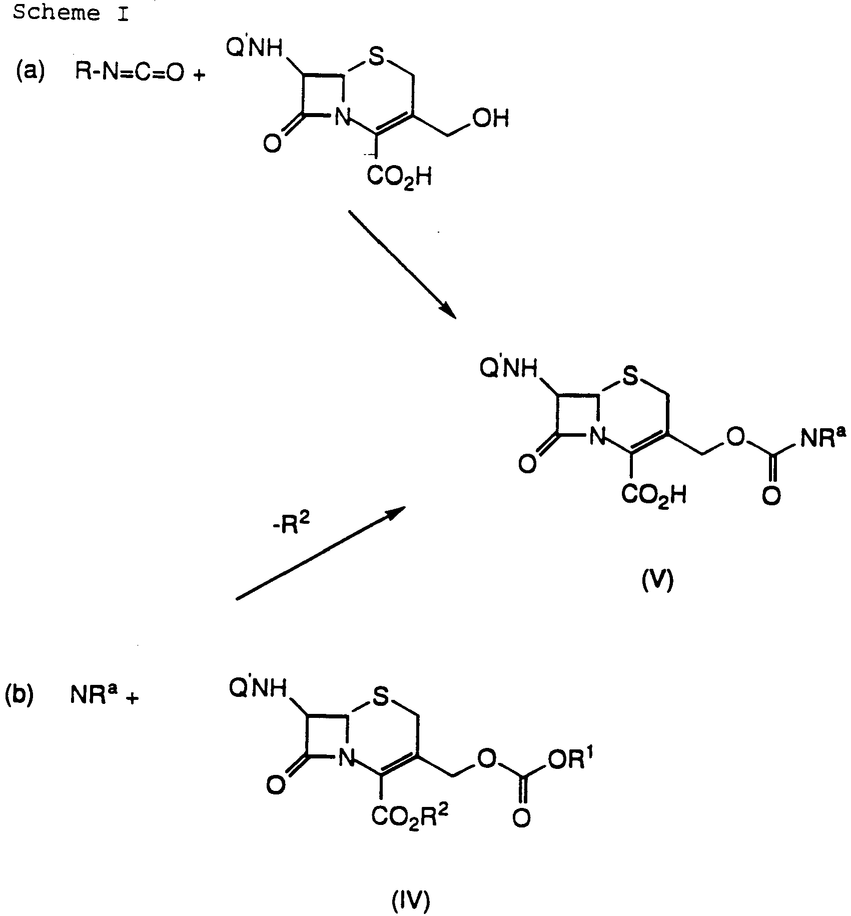 wherein Q' is a protecting group such as 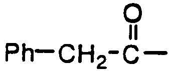 wherein Ph is phenyl.

Compounds of formula (II) before linkage of the polymeric moiety, wherein L is -S-(CH₂)ₙ-and m is 1, may be prepared by a method analogous to route (b) of Scheme I. The preparation of the cephalosporin reactant (VI) and its subsequent elaboration to yield the cephalosporin prodrug of formula (VII) is illustrated in Scheme II. In Scheme II, NR, n, R¹, Q', and R² all have the same meaning as previously defined; X is OH or a halogen atom such as chloro, bromo or iodo. The starting cephalosporin of formula (VI) may be prepared by reacting a carboxyl protected 3-halomethyl cephalosporin of formula (VIII) with a mercaptoalkanol, and the resulting 3-hydroxyalkylthiomethyl cephalosporin is treated with a chloroformate CICO₂R¹, e.g., 4-nitrophenylchloroformate in the presence of a tertiary amine base to afford the compound of formula (VI).

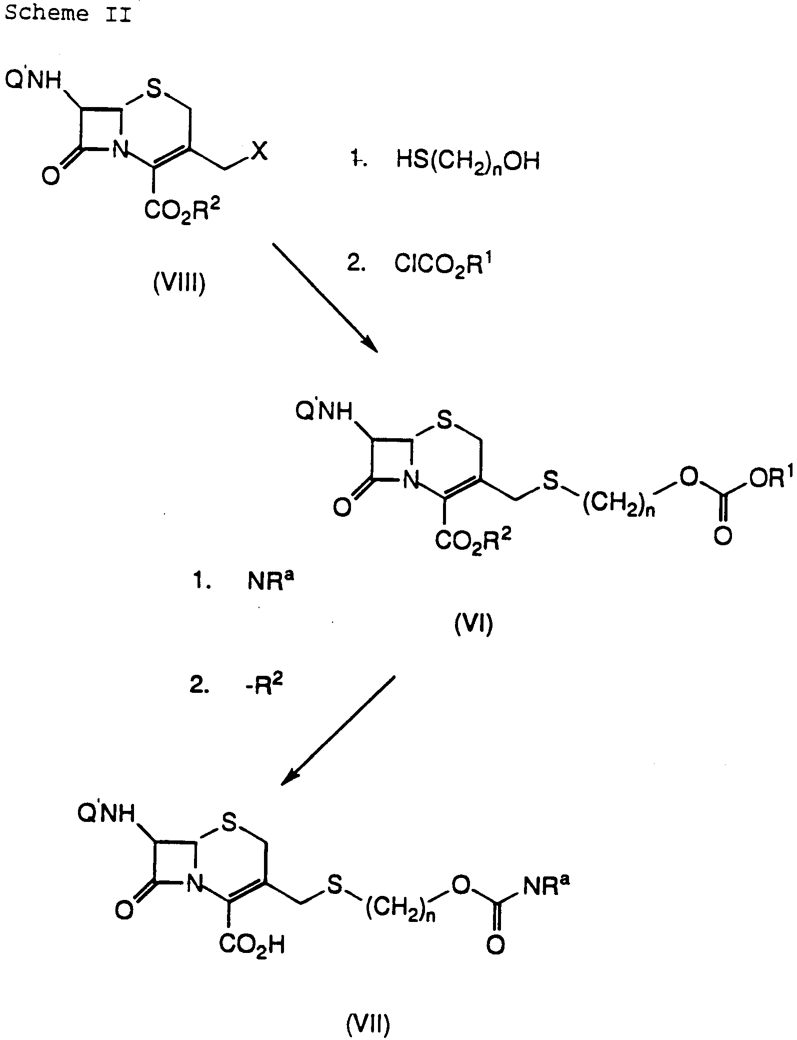

Compounds of formula (II) before linkage of the polymeric moiety, wherein L is -S-(CH₂)ₙ-and m is 0, may be prepared by methods illustrated in Scheme III.

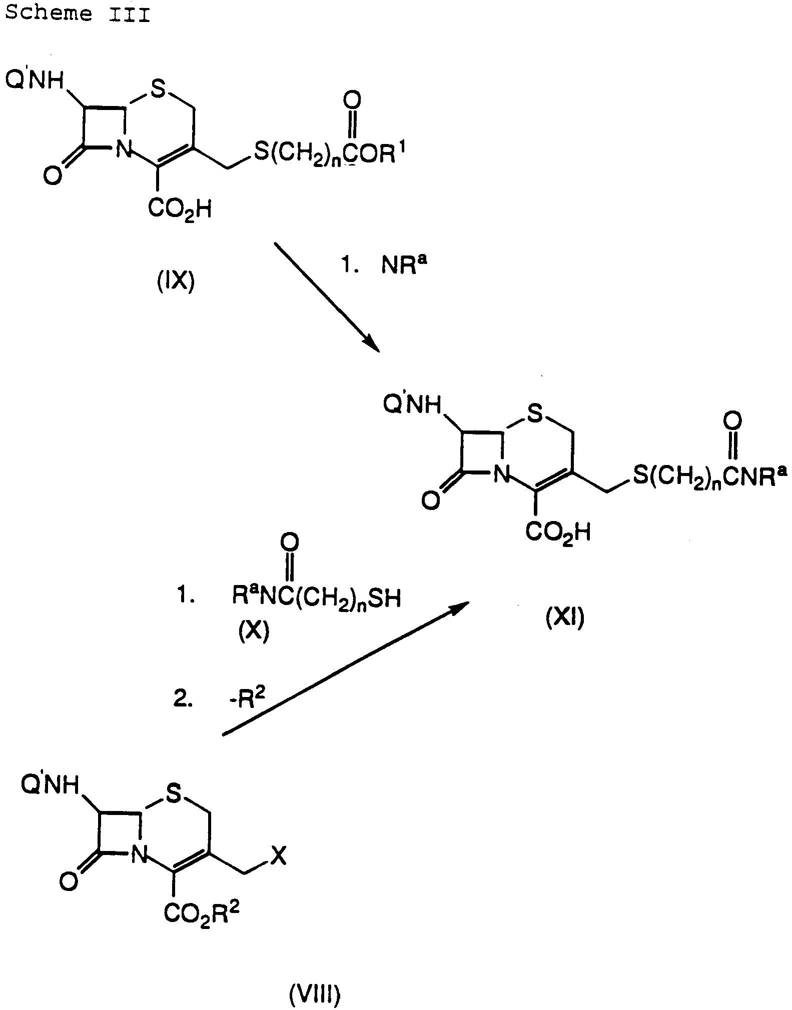

In Scheme III, NR^{a}, n, X, R¹, Q', and R² have the same meaning as previously defined. Thus, a nitrogen containing cytotoxic agent is reacted with a 3-thioalkylcarboxylate substituted cephalosporin derivative (IX) to form the resulting carbamate prodrug of formula (XI). The cephalosporin derivative of formula (IX) in turn may be obtained by reacting a thioalkylcarboxylate, HS(CH₂)ₙCO₂R¹, with a 3-halomethyl cephalosporin, or by reaction a carboxyl protected 3-halomethyl cephalosporin with a thioalkanoic acid followed by activation of the acid moiety. For example, R¹ of compound (IX) may be succinimide or the group -CO₂R¹ may represent a mixed anhydride.

Alternatively, the cytotoxic agent may first be derivatized to form the N-thioalkylcarbonyl compound of formula (X) by reacting NR^{a} with a thioalkylcarboxylate, HS(CH₂)ₙCO₂R¹. Compound (X) is then reacted with a carboxyl-protected 3-halomethyl cephalosporin (VIII) to give the desired product. For the preparation of compounds of formula (XI), R¹ may be, for example, succinimide, or -CO₂R¹ may represent a mixed anhydride).

The cytotoxic drug component NR^{a} may be a member of the nitrogen mustard family as defined above. Particularly preferred mustards are melphalan and N,N-bis(2-chloroethyl)-1,4-benzenediamine (phenylenediamine mustard). In addition, the cytotoxic drug component NR^{a} may be a member of the anthracyline family. Examples of anthracyclines include, but are not limited to, doxorubicin, daunomycin, carminomycin, and the like in which the linkage to the cephalosporin is via the sugar amino group. Preferably, the anthracycline is doxorubicin.

The cytotoxic drug component NR^{a} may also be a member of the mitomycin family. Mitomycins are characterized by the following general structure:

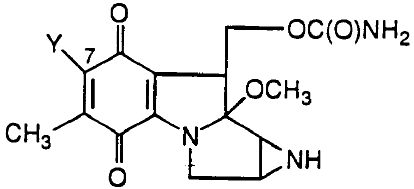

A large number of mitomycin analogs having different substituents on the 7-position have been reported. For the purpose of the present invention, the 7-substituent is not critical as the linkage of the mitomycin to the cephalosporin is through the aziridine nitrogen atom. A preferred mitomycin for the prodrug is mitomycin C, i.e., Y=NH₂. Other examples of mitomycin analogs suitable for the present prodrug may be those disclosed in U.S Patents 4,691,023, 4,803,212, 4,487,769, 4,888,341, and European Published Application 294,828, hereby incorporated by reference.

Linkage of the polymeric moiety is most facilely accomplished after linkage of the cephalosporins to the cytotoxic agent. This can be generally be accomplished by removal of the protecting group (i.e., Q', for example a phenyl acetyl group) from the cephalosporin/drug conjugate using Penicillin G amidase (see, for example, Vrudhula, V.M. et al. J. Med. Chem. (1995), 38, 1380-1385) and reacting the resulting amine with the polymer or modified form (e.g., active ester form) of the polymer. Standard techniques known in the art for reacting amines or otherwise functionalized cephalosporins with polymers are utilized. Techniques for linking other polymers are disclosed in, for example, S. Zalipsky et al., Eur. Polym. J., 19 (12): 1177-1183, 1983; D. Caliceti, et al., II Farmaco, 48 (7): 919-932, 1993; R. Duncan, Anticancer Drugs, 3: 175-210, 1992; and E. F. Panarin et al., J. of Controlled Release, 10: 119-129, 1989.

In another aspect, the present invention provides as a subclass of compounds of formula (I) polymeric cephalosporin prodrugs of the general formula (XII) in which the cytotoxic agent is linked to the cephalosporin nucleus via a carbonate or an ester group 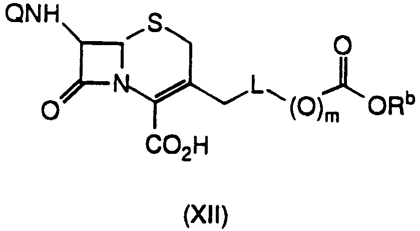 wherein Q, L, and m are as previously defined; OR^{b} is a hydroxy containing cytotoxic drug; or a pharmaceutically acceptable salt thereof. Compounds of formula (XII) may be prepared according to the general methods described in Schemes I to III (with the exception of route (a) in Scheme I) using OR^{b} instead of NR^{a} used therein.

As one example of the present invention, the cytotoxic component OR^{b} is selected from the group of epipodophyllotoxin antitumor agents having the formula 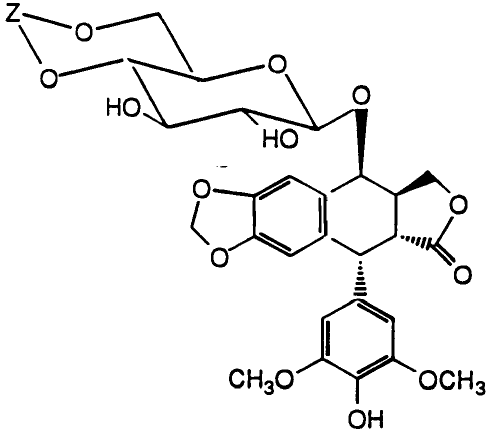 wherein Z is the substituent of a known epipodophyllotoxin glucoside, e.g., alkyl, thienyl, furyl, and phenyl. Particularly preferred are compounds wherein Z is methyl (etoposide) and 2-thienyl (teniposide). These compounds may be linked to the cephalosporin nucleus through the 4'-phenol group.

In another aspect, the present invention provides as a subclass cephalosporin prodrugs of the formula (XIII) 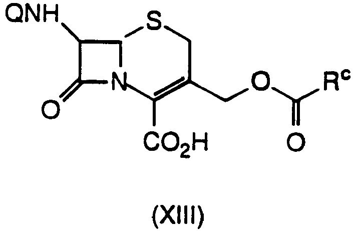 wherein Q is as previously defined; and R^{c}COO is a carboxy containing cytotoxic compound; or a pharmaceutically acceptable salt thereof.

As an example of this, the cytotoxic component melphalan may be linked to the cephalosporin nucleus via the carboxyl group. The melphalan-cephalosporin prodrug (XIV) may be prepared by the procedure depicted in Scheme IV.

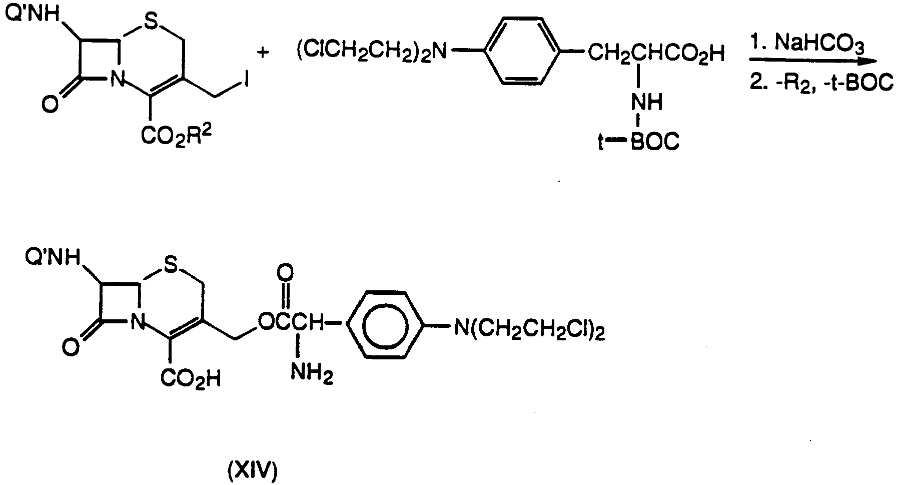

In Scheme IV, Q' and R² are as previously defined. Preferably, R² is an acid labile group such as benzyl or t-butyl. t-BOC is the group t-butoxycarbonyl. Thus, carboxy-protected 3-iodocephalosporin is reacted with N-t-BOC protected melphalan in the presence of a base, e.g., sodium bicarbonate; the resulting diprotected intermediate is treated with an acid to afford the desired product of formula (XIV) and then Q' is replaced with Q as previously described.

Another aspect of the present invention concerns cephalosporin-mitomycin prodrugs having the formula (IIa) 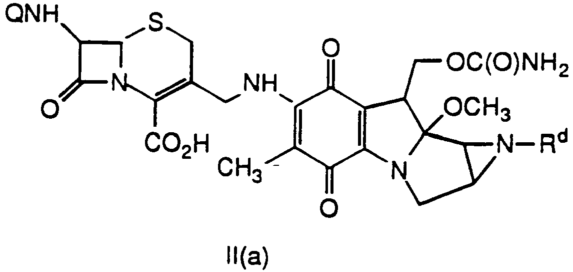 wherein Q and R^{d} are as defined above. Compounds of formula (IIa) are prepared by reacting a 3-aminomethyl cephalosporin with mitomycin A or a N^{1a}-alkyl derivative thereof (N^{1a} refers to the aziridine nitrogen of mitomycins). The reaction is conducted in an organic solvent, e.g. ethanol or methanol, at a temperature conducive to product formation, e g. at ambient temperature. The reaction is generally completed within 24 hours. Preferably, the reaction is carried out under inert atmosphere. The starting material 3-aminomethyl cephalosporin is obtained from the corresponding 3-azidomethyl cephalosporin; both the 3-aminomethyl- and the 3-azidomethyl cephalosporin and methods for their preparation are disclosed in Cocker, J.D. et al, J.Chem. Soc., 1965, 5015 at 5027-5029, the relevant portions thereof are hereby incorporated by reference. The polymeric moiety, Q, is then linked as described previously.

It will be appreciated that synthesis of the polymeric cephalosporin prodrugs encompassed by the present invention is not limited to those procedures and reagents specifically described hereinabove but may be accomplished using other conventional techniques well known to a chemist skilled in the art of organic synthesis. The selection of protecting groups, ester activating groups (and their introduction and removal, if applicable), solvents, and reaction conditions is within the knowledge of a synthetic chemist and may be performed without undue experimentation.

The present invention also encompasses pharmaceutical compositions and methods for treating cancers and other tumors. More particularly, the invention includes compositions comprising polymeric cephalosporin prodrugs which are capable of being cleaved by antibody-β-lactamase conjugates. The prodrugs and enzyme conjugates are used in a method for treating tumors wherein a mammalian host is given a pharmaceutically effective amount of an antibody-enzyme conjugate or conjugates and a pharmaceutically effective amount of a prodrug or prodrugs. The compositions and methods of this invention are useful in treating any mammal, including humans, dogs, cats, and horses.

According to a preferred embodiment, the antibody-enzyme conjugate is administered prior to the introduction of the prodrug into the host. Sufficient time should be allowed between the administration of the conjugate and the prodrug to allow the antibody of the conjugate to target and localize the enzyme to the tumor site. The time may range from 12 hr to one week depending upon the conjugate used.

The conjugates and prodrugs of the invention can be administered using conventional modes of administration including, but not limited to, intravenous, intraperitoneal, oral, intralymphatic, or administration directly into the tumor. Intravenous administration is preferred.

The compositions of the invention may be in a variety of dosage forms which include, but are not limited to, liquid solutions or suspensions, tablets, pills, powders, suppositories, polymeric microcapsules or microvesicles, liposomes, and injectable or infusible solutions. The preferred form depends upon the mode of administration and the therapeutic application. For example, oral administration of the antibody-β-lactamase conjugate may be disfavored because the conjugate proteins tend to be degraded in the stomach if taken orally, e.g., in tablet form.

The conjugate or prodrug compositions also preferably include conventional pharmaceutically acceptable carriers and adjuvants known in the art such as human serum albumin, ion exchangers, alumina, lecithin, buffer substances such as phosphates, glycine, sorbic acid, potassium sorbate, and salts or electrolytes such as protamine sulfate.

The most effective mode of administration and dosage regimen for the compositions of this invention depends upon the severity and course of the disease, the patient's health and response to treatment and the judgment of the treating physician. Accordingly, the dosages of the immunoconjugates and prodrugs should be titrated to the individual patient. Methods of determining dosages are well known in the art. The following examples are to illustrate the invention but should not be interpreted as a limitation thereon.

### Example 1

### 1. Preparation of Polymeric Cephalosporin Prodrug and Biological Studies.

### 1.1 Experimental Procedures

1.1.1 Materials. M-PEG-propionic acid (5 kDa, linear polyethylene glycol polymer capped on one end with a methoxy group) and B-PEG-propionic acid (10 kDa, branched polymer with eight arms projecting from a hydrocarbon core) were obtained from Shearwater Polymers, Inc., Huntsville, AL. Aminocephalosporin doxorubicin (AC-Dox) was prepared from cephalosporin doxorubicin (C-Dox.) and purified as described in U.S. Patent Application Serial Nos. 08/016,208 and 07/609,663 and Vrudhula VM., et al., J. Med. Chem. (1995) 38(8), 1380-1385. The L6 and P1.17 mAbs (both are mouse IgG₂ₐ) and bL from *Enterobacter cloacae* were obtained as previously described in Vrudhula et al., (1991), Bioconjugate Chem. 4, 334-340. L6 binds to antigens present on the H2981 human lung adenocarcinoma cell line, Hellstrom, I et al. (1986), Cancer Res. 46, 3917-3923 The mAb-bL conjugates used were dimeric, and consisted of two mAb F(ab)' fragments attached to bL. These were prepared by reacting a free thiol group on the mAb F(ab)' fragments with maleimide groups appended to bL Svensson, H.P., et al. (1994) Bioconjugate Chem. 5, 262-267. The 3677 cell line was established at Bristol-Myers Squibb, Seattle, WA, from a human metastatic melanoma. Female athymic nu/nu mice (Harlan-Sprague-Dawley, Indianapolis, IN) were injected with 10⁷ cells subcutaneously, and growing tumors were excised and used for subsequent in vivo experiments. Tumor pieces (approximately 2 x 2 mm) were implanted subcutaneously. All in vivo experiments were performed using tumors that had undergone 1-3 in vivo passages. Phosphate buffered saline consists of 10 mM sodium phosphate, 150 mM sodium chloride at pH 7.2.
1.1.2 Preparation of M-PEG-AC-Dox. To a stirred solution of M-PEG propionic acid (10 g, 2 mmol) in 50 mL of CH₂Cl₂ was added 516 mg (2.5 mmol) of dicyclohexylcarbodiimide and 288 mg (2.5 mmol) of N-hydroxysuccinimide. After approximately 12 h, the solids were filtered off, and the filtrate was added dropwise to rapidly stirred ether (approximately 250 mL) at 4 °C. The resulting solid (M-PEG-NHS ester) was collected, dried under high vacuum, and stored at 4 °C until use. The yield of the fine white powder was 9.9 g.
   AC-Dox (0.107 mmol) was added to a freshly prepared solution of M-PEG-NHS ester (1.62 g, 0.32 mmol) in 5 mL of 50 mM borate pH 8.0. After 3.75 h at 37 °C, HPLC analysis indicated that >90% of the AC-Dox was consumed. The reaction mixture was partitioned between saturated aqueous NaCl containing 1% acetic acid, and CHCl₃ containing 20% ethanol. After several such extractions, the combined organic layers were dried (MgSO₄), filtered, and concentrated to dryness. The residue was dissolved in H₂O and stirred with approximately 25 g of Amberlite XAD-7 resin (Sigma Chem. Co., prewashed with CH₃OH followed by H₂O) until the supernatant was almost colorless. The resin was poured onto a 5 x 10 cm bed of prewashed XAD-7 resin, and the resulting column was washed with H₂O (0.5 L), followed by H₂O containing 10% (0.5 L), 20% (0.5 L), 25% (0.2 L) and 80% CH₃OH (0.4 L). The solvent was evaporated from the 80% CH₃OH fraction, and traces of H₂O were azeotropically removed from the residue with toluene. A fine red solid (1.24 g) was obtained by slowly adding a solution of the product in CH₂Cl₂ to rapidly stirred ice cold ether. λₘₐₓ (phosphate buffered saline) 495 nm. ¹H NMR (300 MHz, DMSO-d₆, with suppression of the singlet for OCH₂CH₂O at δ 3.40) d 8.81 (d, 1 H, NH, J_{NH,7} = 8.2 Hz), 7.92 (d, 1 H, ArH, J = 4.8 Hz), 7.65 (m, 2H, ArH), 6.93 (d, 1 H, H-6, J_{6,7} = 8.0 Hz), 5.65 (m, 1 H, H-7), 5.46 (d, 1 H, OH, J = 3.6 Hz), 5.20 (br. s, 1H, H-1"), 5.03 (d, 1H, H-4", J = 4.8 Hz), 4.88 (s, 1 H), 4.60-4.50 (m, 2H, H-14'), 4.13 (m, 1H, H-5"), 3.98 (s, 3H, ArOCH₃), 3.03-2.79 (m, 2H), 2.67 (t, 2H, PEG-COCH₂CH₂, J = 6.0 Hz) 2.20-2.05 (m, 2H, H-8'), 1.90-1.75 (m, 1 H, H-2"A), 1.50-1.40 (m, 1H, H-2"B), 1.11 (d, 3H, H-6", J_{5",6"} = 6.4 Hz).
1.1.3. Preparation of B-PEG-AC-Dox. A solution of B-PEG-propionic acid (1 g, 0.1 mmol) in 10 mL of CH₂Cl₂ containing dicyclohexylcarbodiimide (206 mg, 1 mmol) and N-hydroxysuccinimide (92 mg, 0.8 mmol) was stirred at 23 °C for 4.5 h. The precipitate was filtered and washed with CH₂Cl₂. The filtrate was concentrated to dryness, and the residue was placed under high vacuum and then stored at 4 °C until use. The yield of B-PEG-NHS ester (semi-solid oil) was 1 g.
   To a 37 °C solution of AC-Dox (0.122 mmol) in 10 mL of 0.25 M borate pH 8.0 was added 243 mg (24 mmol) of B-PEG-NHS ester. Additional aliquots (350 mg and 250 mg) of B-PEG-NHS ester were added 3 and 6 h later, respectively. After a total of 10 h at 37 °C, aminoethanol was added (120 mM final concentration) to quench any remaining active esters, and the material was bound to XAD-7 resin as previously described. The resulting XAD-7 column was washed with H₂O (1 L), and then with H₂O containing 25% (0.5 L), 37% (0.5 L), 50% (0.25 L), and 75% (0.5 L) CH₃OH. Polymer-bound drug was mainly contained in the 75% CH₃OH fraction. This fraction was concentrated to dryness, dissolved in CH₂Cl₂ containing 5% CH₃OH, and extracted with saturated aqueous NaCl containing 1% acetic acid. After several back extractions of both layers, the combined organic phases were dried (MgSO₄), filtered, and concentrated to dryness. The red residue was lyophilized from H₂O to give 439 mg of B-PEG-AC-Dox as a dark red powder. λₘₐₓ (phosphate buffered saline) 485 nm. ¹H NM R (300 MHz, DMSO-d₆, with suppression of the singlet for OCH₂CH₂O at d 3.40) d 8.90-8.80 (m, 1 H, NH), 8.27 (d, 1 H, ArH, J = 7.8 Hz), 7.97-7.89 (m, 2H, ArH), 6.92 (d, 1H, H-6, J = 5.7 Hz), 6.02-5.58 (m, 1 H), 5.45 (d, 1H, OH, J = 4.5 Hz), 5.21 (br. s, 1H, H-1 "), 4.92 (br s., 1 H), 4.62-4.50 (m, 2H, H-14'), 4.20-4.04 (m, 1 H, H-5"), 3.98 (s, 3H, ArOCH₃), 2.90-2.82 (m, 2H, PEG-COCH₂CH₂).
1.1.4. Kinetic Studies. The kinetic parameters for the hydrolyses of 5-100 mM solutions of either M-PEG-AC-Dox or B-PEG-AC-Dox (in phosphate buffered saline containing 12.5 mg/mL bovine serum albumin) with bL (0.2 µg/mL for M-PEG-AC-Dox, 1.0 µg/mL for B-PEG-AC-Dox) were determined spectrophotometrically by determination of the initial velocities of the reactions as a function of substrate concentration. Lineweaver-Burk plots of the data were used to estimate the Kₘ and Vₘₐₓ values. Cleavage of the p-lactam ring resulted in a loss of absorbance at 260 nm for both M-PEG-AC-Dox (Δε₂₆₀ nm = -7.9 X 10⁻³ M⁻¹ cm⁻¹) and B-PEG-AC-Dox (Δε₂₆₀ nm = -3.8 X 10⁻³ M⁻¹ cm⁻¹).
1.1.5. In Vitro Cytotoxicity Assays. H2981 cells were grown in monolayer cultures in Iscove's modified Dulbecco's medium supplemented with 10% (v/v) fetal bovine serum, 0.1 mg/mL streptomycin, and 0.06 mg/ml penicillin G, plated out into 96-well plates (10,000 cells/well for M-PEG-AC-Dox, 5,000 cells/well for B-PEG-AC-Dox), and allowed to adhere for 18 h at 37 °C. The plates were washed with Roswell Park Memorial Institute 1640 medium RPMI, antibiotic free), mAb-bL conjugates were added (3.16 µg mAb component/mL for M-PEG-AC-Dox, 1 µg/mL mAb component for B-PEG-AC-Dox), and incubation was continued for 30 min at 4 °C. The cells were washed three times with RPMI, treated with prodrug (1h for M-PEG-AC-Dox, 2 h for B-PEG-AC-Dox) at 37 °C, washed, and incubated for 18 h at 37 °C. [³H]-Thymidine (1 µCi) was added to each well, and the cells were harvested and counted after 5 h (for M-PEG-AC-Dox) or 4 h (for B-PEG-AC-Dox). The results were compared to untreated cells, cells that were saturated with L6 (1 mg/mL) prior to conjugate exposure, and to non-conjugate treated cells that were treated with doxorubicin instead of PEG-AC-Dox.
1.1.6. Pharmacokinetics. All experiments were performed in athymic female nu/nu mice (Harlan-SpragueDawley, Indianapolis, IN) and were initiated when the animals reached an age of 6-10 weeks. 3677 human tumor xenografts from in vivo passaging were implanted subcutaneously and allowed to grow to approximately 200 mm³. B-and M-PEG-AC-Dox (freshly prepared at 5.2 mM in phosphate buffered saline) were injected (intraveneously) so that each mouse received 52 µmol PEG-AC-Dox/kg body weight. Doxorubicin (1.55 mM in saline) was injected so that each mouse received 15.5 µmol doxorubicin/kg (9 mg/kg). At various time points, the mice (3 animals/group) were anesthetized, bled via the retro-orbital plexus, and then euthanized. Tumors were rapidly excised, frozen in liquid N₂, and stored at -70 °C. Heparinized blood samples were centrifuged, and the plasma was frozen at -70°C.
   Methanol (0.5 mL) was added to 50 mL aliquots of freshly thawed plasma samples. The samples were sonicated and centrifuged at 10,000 g for 3 min. The supernatants were evaporated to dryness under reduced pressure, and the residues were dissolved in 50 mL of 20% acetonitrile in 50 mM triethylammonium formate pH 2.8 for HPLC analyses. Tumor samples (approximately 200 mg/sample) were rapidly thawed, weighed, and homogenized in 0.5 mL of methanol. Solids were removed by centrifugation (10,000 *g* for 3 min), and the supernatants were evaporated to dryness under reduced pressure. The residues were dissolved in 50 µL of 20% acetonitrile in 50 mM triethylammonium formate pH 2.8 for HPLC analyses. Standard curves were obtained by extracting tumor and plasma samples containing known amounts of doxorubicin or the PEG-AC-Dox polymers.
1.1.7. HPLC Analysis. Two different HPLC analytical systems were used for the PEG-AC-Dox preparations. A Beckman HPLC system was used to characterize the different preparations to confirm that doxorubicin was released upon addition of bL, and to measure the stability of the polymer preparations in phosphate buffered saline and mouse plasma. Analyses were performed using a Phenomenex 4 x 150 mm C-18 reversed phase column, detection at 495 nm, and a flow rate of 1 mL/min. A linear gradient of 20-80% CH₃CN in 50 mM triethylammonium formate buffer at pH 2.8 was employed as the eluting solvent. A Hewlett Packard 1090 HPLC system with a refrigerated auto sampler and a diode array detector was used to determine plasma and intratumoral PEG-AC-Dox and doxorubicin concentrations. Separation of samples was achieved using a 2.1 x 250 mm Spheri-5 RP-18 column (Applied Biosystems/Brownlee) equipped with a matching guard column. Samples were eluted and detected as described above, but at a flow rate of 0.2 mL/min. Concentrations were determined by integration of the absorbance peaks and comparision to standard curves obtained from tumor and blood samples having known amounts of added doxorubicin, M-PEG-AC-Dox, or B-PEG-AC-Dox.
1.1.8. Stability Studies. Solutions of the PEG-AC-Dox samples at 0.5 mM were prepared in phosphate buffered saline or mouse plasma and incubated at 37 °C. At periodic intervals, samples were subjected to HPLC analysis as described above. The mouse plasma samples were diluted with one volume of methanol, and the precipitated proteins were separated by centrifugation. The resulting supernatants were analyzed by HPLC.

### 1.2 RESULTS

1.2.1. Preparation and Characterization of PEG-AC-Dox Derivatives. 7-Aminocephalosporin doxorubicin (AC-Dox, Chart 1) was prepared by enzymatic hydrolysis of 7-phenylacetylcephalosporin doxorubicin (C-Dox, Hudyma, T.W., et al. (1993) Bioorg. Med. Chem. Lettrs., 3, 323-328) with penicillin-G amidase as previously described in U.S. Patent Application Serial No. 07/609,663, Purification was achieved using reversed phase chromatography. Condensation of AC-Dox with the N-hydroxysuccinimide esters of monomethoxy-PEG-propionic acid (MW 5 kDa) or branched-PEG-propionic acid (MW 10 kDa) led to the formation of M-PEG-AC-Dox and B-PEG-AC-Dox, respectively. Branched-PEG-propionic acid is an octomeric form of PEG that is prepared by ethoxylation of a polyalcohol. A two-step purification procedure was used to separate the desired product from unconjugated doxorubicin, doxorubicin derivatives, and PEG. Unmodified polymer was removed chromatographically on a column of polyacrylic ester resin (Amberlite XAD-7) using a stepwise gradient of methanol for compound elution. Under these conditions, free polymer eluted off the column before PEG-AC-Dox. Further purification was achieved by extracting the polymer solution with weak acid to remove amine-containing impurities.
The ¹H-NMR spectra (300 MHz) for the PEG-AC-Dox derivatives revealed the expected protons for PEG, cephalosporin, and doxorubicin, and were therefore consistent with the structures shown in Chart 1 below.

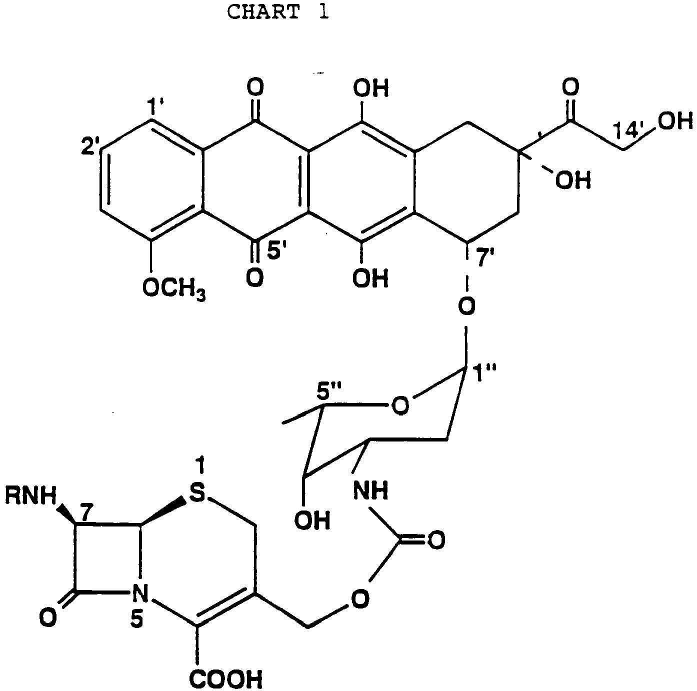

| R | Compound |
|---|---|
| C₆H₅CH₂CO | C-Dox |
| H | AC-Dox |
| M-PEG-OCH₂CH₂CO | M-PEG-AC-Dox |
| B-PEG-OCH₂CH₂CO | B-PEG-AC-Dox |

Integration of the peaks indicated that the PEG to doxorubicin molar ratios for both polymers were approximately one. The polymers were further characterized by reversed phase HPLC, in which M-PEG-AC-Dox (Figure 2A) and B-PEG-AC-Dox (Figure 2B) eluted primarily as single peaks. The HPLC profile for the B-PEG-AC-Dox used in the studies reported here (Figure 2B) indicates the presence of some free doxorubicin (1.2% of the total integrated value). More recent preparations of B-PEG-AC-Dox were free of any detectable unbound doxorubicin (data not shown). Reaction of the polymers with bL led to the formation of doxorubicin, based on comparison with an authentic sample (Figures 2C and 2D). These studies provide evidence for the structural assignments, and indicate that free doxorubicin can be released from the PEG-AC-Dox polymers upon bL catalyzed hydrolysis.
A spectophotometric method was used to quantify the rate of b-lactam catalyzed hydrolysis for each of the polymers. The kinetic constants, derived from Lineweaver-Burk plots, were compared to those obtained for the hydrolysis of C-Dox (Table 1). The turnover rates with M-PEG-AC-Dox and B-PEG-AC-Dox as substrates for bL were 8-13 times slower than with C-Dox as substrate. In contrast, the Km values for the three substrates were similar. Thus, the polymers and C-Dox apparently bind equally well to bL, but undergo hydrolysis at differing rates.
An HPLC assay was used to determine the stabilities of M-PEG-AC-Dox and B-PEG-AC-Dox in phosphate buffered saline and mouse plasma. After 24 h in phosphate buffered saline at 37 °C, both polymers underwent a small amount of hydrolysis resulting in the release of approximately 10% of the bound doxorubicin. Incubation of M-PEG-AC-Dox and B-PEG-AC-Dox in mouse plasma at 37 °C led to the release of 26% and 7% of doxorubicin, respectively. Thus, under the test conditions, most of the doxorubicin remains covalently attached to the polymer support.

**TABLE 1**

| Kinetic Constants for bL^{a} | | | |
|---|---|---|---|
| substrate | Vmax (µmol/mim /mg | kcat (sec⁻¹) | Km (µM) |
| | | | |
| M-PEG-AC-Dox | 46 ± 11 | 33 ± 8 | 34 ± 13 |
| B-PEG-AC-Dox | 37 ± 10 | 28 + 6 | 43 + 12 |
| C-Dox | 400 ± 96 | 280 ± 67 | 46 ± 6 |

| | | | |
|---|---|---|---|
| ^{a} The values wre obtained from 3-4 independently run experiments. | | | |

1.2.2 In Vitro Cytotoxic Activities. The cytotoxic effects of M-PEG-AC-Dox, B-PEG-AC-Dox, doxorubicin, and combinations of the mAb-bL conjugates with the polymer preparations were determined on the H2981 human lung adenocarcinoma cell line. Several experiments were performed, and the conditions described represent those that led to the highest degrees of immunologically specific prodrug activation and cytotoxic differentials between prodrug and drug. The bivalent [F(ab)']₂-bL conjugates used were prepared by combining maleimide substituted bL with L6 F(ab')-SH as previously described Svensson, H.P., et al.,(1994), Bioconjugate Chem. 5, 262-267. This conjugation procedure does not alter mAb binding or the enzymatic activity of bL Svensson, H.P., et al.,(1994), Bioconjugate Chem. 5, 262-267.
H2981 cells were exposed to the polymers (1 h for M-PEG-AC-Dox, 2 h for B-PEG-AC-Dox), washed, and after further incubation, toxicity was determined by measuring the incorporation of [³H] thymidine into DNA. The cytotoxic activities of M-PEG-AC-Dox (IC₅₀ = 80 µM) and B-PEG-AC-Dox (IC₅₀ = 8 µM) were much lower than that of doxorubicin (IC₅₀ = 0.1-0.2 µM, Figure 3). There is probably no significance in the 10-fold difference in IC₅₀ values between the two polymers, since they were tested under different conditions and B-PEG-AC-Dox contained a small amount of free doxorubicin (1.2%) at the beginning of the assay (Figure 2B). A significant increase in polymer cytotoxicity was obtained on cells that were pretreated with L6-bL. It is noteworthy that the L6-bL + PEG-AC-Dox combinations did not yield the same level of cytotoxic activity as doxorubicin. This is most likely due to incomplete drug release under conditions of the assay, since higher degrees of activation were obtained by not washing off unbound conjugate or by adding high concentrations of free bL to the polymer samples.
Activation of the polymers took place in an immunologically specific manner as evidenced by the fact that the cytotoxic activities of the polymers were not increased on H2981 cells that were saturated with unmodified L6 mAb prior to exposure to L6-bL. In addition, P1.17-bL did not effect the activation of B-PEG-AC-Dox on H2981 cells (P1.17 antigen negative). Taken together, these studies show that both M-PEG-AC-Dox and B-PEG-AC-Dox are relatively non-cytotoxic doxorubicin prodrugs that can be activated by L6-bL on cells that are L6 antigen positive.
1.2.3 In Vivo Studies. The toxicities of the polymer preparations in nude mice were compared to that of doxorubicin. The maximum tolerated dose (defined as the drug dose that gave < 20% weight loss, no deaths, and was within 30% of the dose where > 20% weight loss and/or deaths occurred) of doxorubicin after a single intravenous injection was 13.8 µmol/kg body weight (8 mg/kg). Although the maximum tolerated doses for the polymers have not yet been determined, it was possible to safely administer 52 µmol/kg of M-PEG-AC-Dox and B-PEG-AC-Dox with no weight loss or long-term toxicities. Some animals experienced considerable discomfort immediately after treatment with the polymers. This was most likely due to the non-isotonic nature of the injected solutions, since equivalent doses of free PEG elicited the same immediate response. Intraperitoneal injection of the polymers circumvented this problem, but subsequent studies were performed using the intravenous route, since this is the usual mode of administration for doxorubicin.
Tumor uptake and blood clearance studies were undertaken in nude mice bearing subcutaneous 3677 human melanoma tumors. At a point when the tumors were approximately 200 mm³ in volume, the animals were given intravenous injections of doxorubicin (15.5 µmol/kg), M-PEG-AC-Dox (52 µmol/kg), or B-PEG-AC-Dox (52 µmol/kg). Blood and tumors were removed at various time points, and an HPLC assay was used to determine the concentrations of the injected materials in the extracted samples. Calibration curves were obtained from several tumors and blood samples that were spiked with varying concentrations of free doxorubicin, M-PEG-AC-Dox, or B-PEG-AC-Dox. None of the extracted samples contained detectable levels of AC-Dox, indicating that the amide bond between the cephalosporin 7-amino group and the terminal carboxyl group on PEG is stable.
The blood levels of B-PEG-AC-Dox were higher than M-PEG-AC-Dox for all of the time points tested (Figure 4A). This is due most likely to slower blood clearance of B-PEG-AC-Dox compared to M-PEG-AC-Dox. The highest level of plasma doxorubicin measured, 0.64 nmol/g at 15 min, was substantially lower than the polymer levels. The doxorubicin levels in Figure 4A are consistant with a previously reported phar-macokinetic study which showed that doxorubicin had an initial half life of 2 min in mice Bosslet, K.,etal., (1994), Cancer Res. 54, 2151-2159.
Thus, it was not surprising that no free doxorubicin was detected in plasma obtained from mice that were treated with the PEG-AC-Dox polymers. In addition to having a longer plasma half life, B-PEG-AC-Dox was retained in 3677 tumors to a greater extent than was M-PEG-AC-Dox (Figure 4B). The highest intratumoral level of B-PEG-AC-Dox was obtained 30 min after injection, and substantial levels were still present 4 h post-injection. Over the 4 h measurement period, the area under the curve for B-PEG-AC-Dox was 2.1-fold greater than that of M-PEG-AC-Dox. It is noteworthy that the intratumoral doxorubicin levels were quite low in mice that received doxorubicin. The maximum doxorubicin level, 1.41 nmol/g at 30 min post-injection, was much lower than the polymer concentrations at all time points tested. Thus, both polymers deliver substantially greater amounts of intratumoral doxorubicin equivalents over an extended period of time compared to systemic doxorubicin treatment.

Thus, novel polymeric cephalosporin prodrugs and methods for their use have been disclosed. Although the preferred embodiments of the subject invention have been described in some detail, it is understood that obvious variations can be made without departing from the spirit and the scope of the invention as defined by the appended claims.

## Claims

1. A polymeric cephalosporin prodrug having the formula 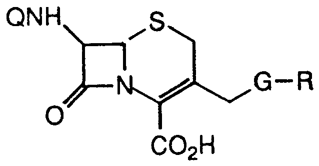 wherein
Q is a pharmaceutically acceptable polymeric moiety,G is -NH- or is of the formula 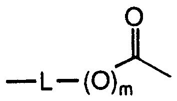
L is a direct bond or -S-(CH₂)ₙ-;
R is an agent capable of exerting a cytotoxic effect on tumor cells when released from said cephalosporin prodrug;
n is 2, 3 or 4; and
m is 0 or 1 with the proviso that, when L is a direct bond, m is 1; or
a pharmaceutically acceptable salt thereof.

2. A polymeric cephalosporin prodrug of claim 1 having the formula 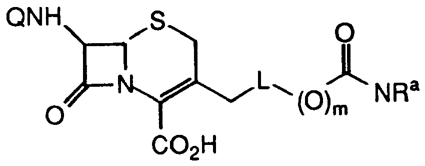 wherein NR^{a} is a nitrogen containing cytotoxic agent; Q,L,and M are as defined in Claim 1; or a pharmaceutically acceptable salt thereof.

3. The polymeric cephalosporin prodrug of claim 1, wherein R is selected from the group consisting of etoposide, teniposide, doxorubicin, daunomycin, carminomycin, aminopterin, nitrogen mustard, dactinomycin, mitomycin, cis-platinum and cis-platinum analogues, bleomycins, esperamicins, and 5-fluorouracil.

4. The polymeric cephalosporin prodrug of claim 2, wherein NR^{a} is a nitrogen mustard, mitomycin C or doxorubicin.

5. The polymeric cephalosporin prodrug of claim 2 having the formula: 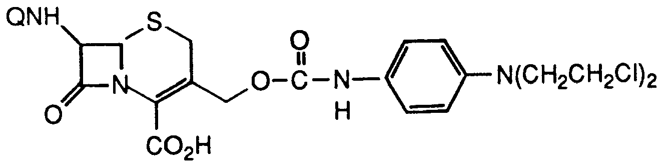 wherein Q is as defined in claim 1; or a pharmaceutically acceptable salt thereof.

6. The polymeric cephalosporin prodrug of claim 1 wherein Q is a polymeric residue of a compound selected from the group consisting of a polyethylene glycol, a branched polyethylene glycol, a polyethylene glycol copolymer, dextran, hydroxypropylmethacrylamide, poly(styrene-co-maleic acid), polygalacturonic acid, carboxymethylated β-D-glucan, N-(2-hydroxypropyl)methacrylamide copolymer, N-(2-hydroxyethyl)methacrylatevinyl-pyrrolidone copolymer, poly(hydroxyethyl-L-glutamine), poly(α-maleic acid), a polyamino acid, and a block copolymer of poly(ethyle-neimine) and palmitic acid.

7. The polymeric cephalosporin prodrug of claim 1 wherein Q is an optionally modified polymeric residue of a polyethylene glycol compound having the formula
R'-(OCH₂-CH₂)ₙ-OH
wherein R' is H or C₁-C₄ alkyl and n is an integer of 4 to 1000.

8. The polymeric cephalosporin prodrug of claim 7 wherein R' is H or methyl and n is an integer of 10 to 500 or 50 to 200.

9. The polymeric cephalosporin prodrug of claim 1 wherein Q is an optionally modified polymeric residue of a branched polyethylene glycol compound having the formula
R"-[(OCH₂CH₂)ₙ-OH]ₓ
wherein R" is a hydrocarbon backbone containing 2 to 12 carbon atoms that is the residue of a polyalcohol, n is an integer of 4 to 500, and x is an integer of 2 to 12.

10. The polymeric cephalosporin prodrug of claim 9 wherein n is an integer of 10 to 400 or 50 to 200 and x is an integer of 4 to 10.

11. The polymeric cephalosporin prodrug of claim 2 having the formula: 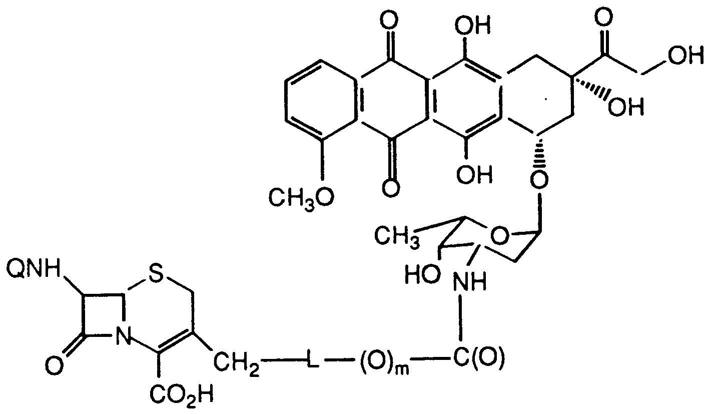 wherein Q is a polymeric residue of an optionally modified polyethylene glycol, or an optionally modified branched polyethylene glycol; and L and m are as defined in claim 1; or a pharmaceutically acceptable salt thereof.

12. The polymeric cephalosporin prodrug of claim 2 having the formula: 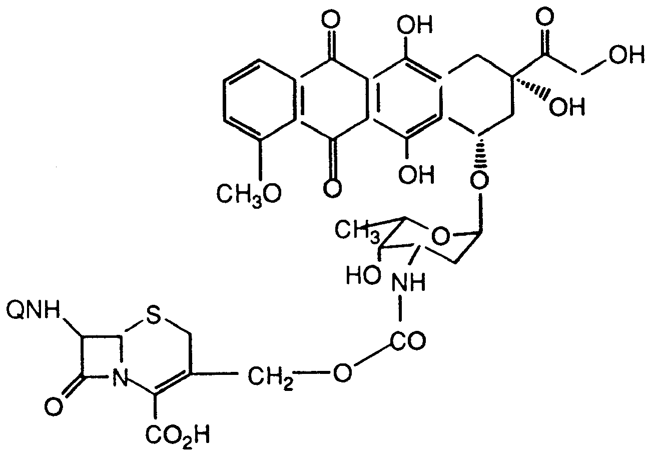 wherein Q is a polymeric residue of an optionally modified polyethylene glycol, or an optionally modified branched polyethylene glycol; or a pharmaceutically acceptable salt thereof,
wherein the residue of an optionally modified polyethylene glycol has the formula
R'-(OCH₂-CH₂)ₙ-OH
wherein R' is H or C₁-C₄ alkyl and n is a integer of 10 to 500, and the residue of an optionally modified branched polyethylene glycol has the formula:
R"-[(OCH₂CH₂)ₙ-OH]ₓ
wherein R" is a hydrocarbon backbone containing 2 to 12 carbon atoms that is the residue of a polyalcohol, n is an integer of 10 to 400, and x is an integer of 4 to 10

13. The polymeric cephalosporin prodrug of claim 2 having the formula 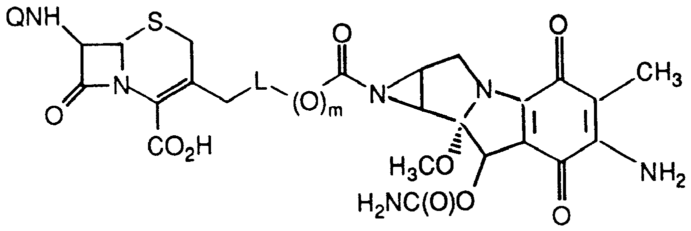 wherein Q is the polymeric residue of an optionally modified polyethylene glycol, or an optionally modified branched polyethylene glycol; and L, m are as defined in claim 1; or a pharmaceutically acceptable salt thereof,
wherein the residue of an optionally modified polyethylene glycol has the formula
R'-(OCH₂-CH₂)ₙ-OH
wherein R' is H or methyl and n is a integer of 10 to 500, and the residue of an optionally modified branched polyethylene glycol has the formula:
R"-[(OCH₂CH₂)ₙ-OH]ₓ
wherein R" is a hydrocarbon backbone containing 2 to 12 carbon atoms that is the residue of a polyalcohol, n is an integer of 10 to 500, and x is an integer of 4 to 10.

14. The polymeric cephalosporin prodrug of claim 2 having the formula: 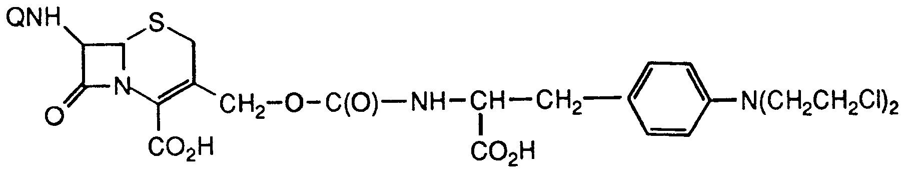 wherein Q is the polymeric residue of an optionally modified polyethylene glycol, or an optionally modified branched polyethylene glycol; or a pharmaceutically acceptable salt thereof,
wherein the residue of an optionally modified polyethylene glycol has the formula
R'-(OCH₂-CH₂)ₙ-OH
wherein R' is H or methyl and n is a integer of 10 to 500, and the residue of an optionally modified branched polyethylene glycol has the formula:
R"-[(OCH₂CH₂)ₙ-OH]ₓ
wherein R" is a hydrocarbon backbone containing 2 to 12 carbon atoms that is the residue of a polyalcohol, n is an integer of 10 to 500, and x is an integer of 4 to 10.

15. The polymeric cephalosporin prodrug of claim 2 having the formula: 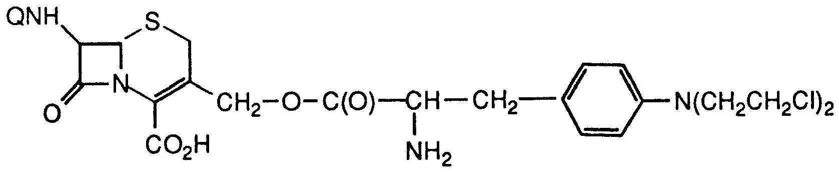 wherein Q is the polymeric residue of a polyethylene glycol, or a branched polyethylene glycol; or a pharmaceutically acceptable salt thereof,
wherein the residue of an optionally modified polyethylene glycol has the formula
R'-(OCH₂-CH₂)ₙ-OH
wherein R' is H or methyl and n is a integer of 10 to 500, and the residue of an optionally modified branched polyethylene glycol has the formula:
R"-[(OCH₂CH₂)ₙ-OH]ₓ
wherein R" is a hydrocarbon backbone containing 2 to 12 carbon atoms that is the residue of a polyalcohol, n is an integer of 10 to 500, and x is an integer of 4 to 10.

16. A polymeric cephalosporin prodrug having the formula: 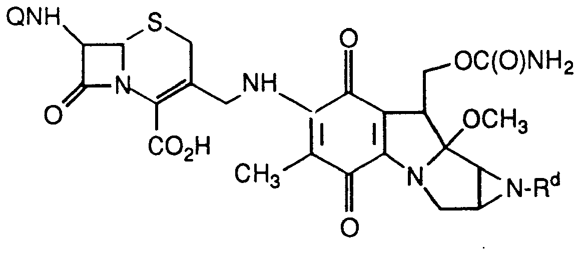 wherein R^{d} is hydrogen or C₁₋₃ alkyl, and Q is the polymeric residue of an optionally modified polyethylene glycol, or an optionally modified branched polyethylene glycol; or a pharmaceutically acceptable salt thereof.

17. The polymeric cephalosporin prodrug of claim 16 wherein R^{d} is hydrogen and Q is the residue of an optionally modified polyethylene glycol of the formula
R'-(OCH₂-CH₂)ₙ-OH
wherein R' is H or methyl and n is a integer of 10 to 500, or the residue of an optionally modified branched polyethylene glycol of the formula:
R"-[(OCH₂CH₂)ₙ-OH]ₓ
wherein R" is a hydrocarbon backbone containing 2 to 12 carbon atoms that is the residue of a polyalcohol, n is an integer of 10 to 500, and x is an integer of 4 to 10.

18. The use of at least one antibody-β-lactamase conjugate and of at least one prodrug according to any of claims 1-17 for preparing a pharmaceutical composition for treating mammalian tumors.

19. A method for the delivery of a cytotoxic agent to tumor cells comprising:
administering a pharmaceutically effective amount of at least one antibody-β-lactamase conjugate wherein
said antibody is reactive with an antigen on the surface of said tumor cells; and
administering a pharmaceutically effective amount of a polymeric cephalosporin prodrug of any of claims 1-17.
whereby said cytotoxic agent is delivered to said tumor cell.

20. The method of claim 19, wherein said antibody is selected from the group consisting of polyclonal, monoclonal, or chimeric antibodies, and said cytotoxic agent is selected from the group consisting of the doxorubicin, mitomycin C, and nitrogen mustards.

21. The method of claim 20, wherein said polymeric cephalosporin prodrug has the general formula 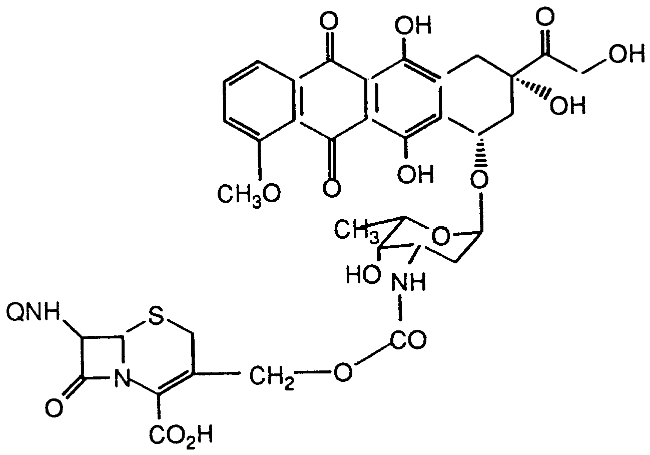 where Q is the residue of an optionally modified polyethylene glycol or an optionally modified branched polyethylene glycol; or a pharmaceutically acceptable salt thereof,
wherein the residue of an optionally modified polyethylene glycol has the formula
R'-(OCH₂-CH₂)ₙ-OH
wherein R' is H or methyl and n is a integer of 10 to 500, and the residue of an optionally modified branched polyethylene glycol has the formula:
R"-[(OCH₂CH₂)ₙ-OH]ₓ
wherein R" is a hydrocarbon backbone containing 2 to 12 carbon atoms that is the residue of a polyalcohol, n is an integer of 10 to 500, and x is an integer of 4 to 10.

22. The method of claim 20, wherein said polymeric cephalosporin prodrug is 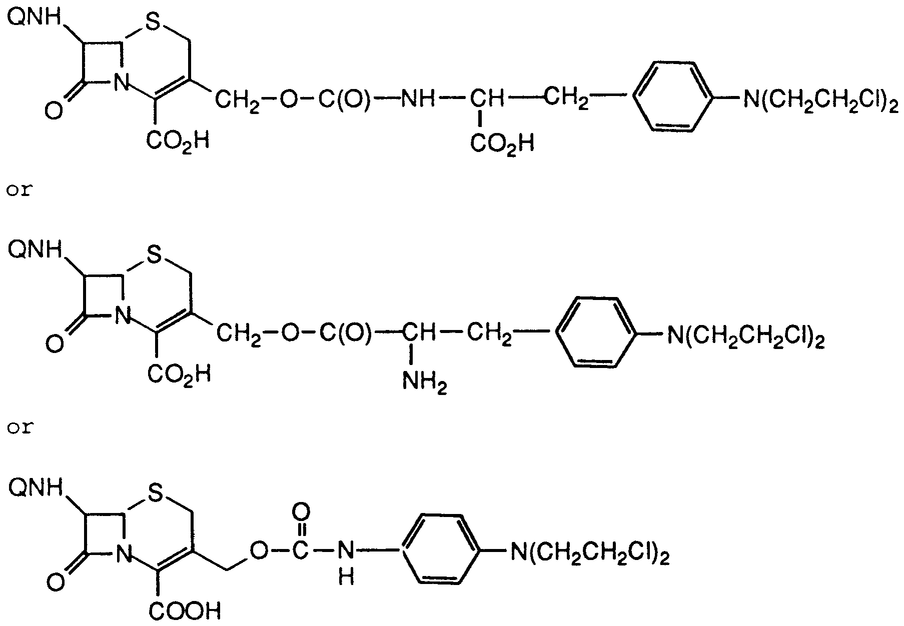 where Q is a residue of an optionally modified polyethylene glycol or an optionally modified branched polyethylene glycol; or a pharmaceutically acceptable salt thereof.

23. The method of claim 20 wherein said polymeric cephalosporin prodrug has the formula 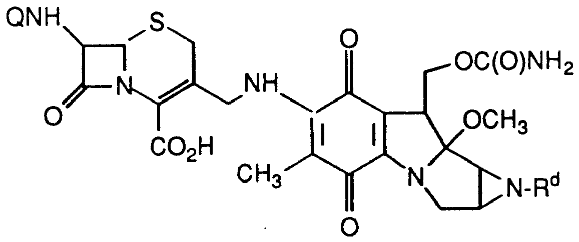 wherein R^{d} is hydrogen or C₁₋₃ alkyl, and Q is the residue of an optionally modified polyethylene glycol or an optionally modified branched polyethylene glycol; or a pharmaceutically acceptable salt thereof.

24. A method for the delivery of a cytotoxic agent to tumor cells comprising:
administering a pharmaceutically effective amount of at least one fusion protein comprising at least the antigen binding region of an antibody reactive with a tumor-associated antigen linked to at least a functionally active part of β-lactamase; and
administering a pharmaceutically effective amount of a polymeric cephalosporin prodrug.

25. A pharmaceutical composition comprising at least one polymeric cephalosporin prodrug of any of claims 1 to 17, optionally together with conventional pharmaceutically acceptable carriers and adjuvants.

26. The composition of claim 25 which additionally comprises at least one antibody-β-lactamase conjugate.

27. The composition of claim 26 in the form of a kit of parts in which the first part comprises said prodrug and the second part comprises said conjugate.
